# EUROPEAN PATENT APPLICATION

(11) **EP 4 435 102 A1**
(43) Date of publication of application: **25.09.2024**
(21) Application number: 23163903.0
(22) Date of filing: 24.03.2023
(51) Int. Cl.: C12N 15/10, C07C 323/60, C07D 487/04, C07D 495/04, C12N 5/00, C12N 15/01, C12N 15/62, C12N 15/75, G01N 33/50, G01N 33/68

(54) **SCREENING METHOD**

(71) Applicant: AB Enzymes GmbH, 64293 Darmstadt (DE)
(72) Inventor: SEEFRIED, Michael, 64293 Darmstadt (DE); WALGRAEVE, Jonathan, 64293 Darmstadt (DE)
(74) Representative: Espatent Oy

(57) **Abstract**

A screening method for cells having increased production of a thiol-containing compound, the method comprising the steps of: (i) providing cells producing the thiol-containing compound, (ii) cultivating the cells separately from each other in the presence of a sulfhydryl reagent, and (iii) selecting cells exhibiting increased progression of cell cycle when compared to the rest of the cultivated cells, thereby obtaining a subgroup comprising cells having increased production of the thiol-containing compound.

## Description

### TECHNICAL FIELD

The present disclosure generally relates to a screening method of cells for production of a thiol-containing compound. The disclosure relates particularly, though not exclusively, to a screening method for cells exhibiting increased progression of cell cycle, when cultured under sulfhydryl reagent pressure.

### BACKGROUND

This section illustrates useful background information without admission of any technique described herein representative of the state of the art.

As producing compounds, such as proteins in selected host cell systems becomes more and more important in fields such as commercial food and chemical production, as well as in medical therapeutics, there is a need to provide increased protein yield from various cell types, including both prokaryotic and eukaryotic cells. Therefore, improving the production level of wild type compounds, as well as compounds, such as proteins (including, but not being limited to, non-catalytical proteins), metabolites, peptides and even free amino acids obtained through recombinant genetic modification, is pursued both by academia, as well as commercial sectors. Many of such compounds of interest comprise thiol groups.

Traditionally, the commonly used methods for screening the candidate cell clones producing the highest levels of compound of interest, require expanding cultures and adapting each of the clones to culture conditions, which is resource- and time demanding. Therefore, even though developments have been made in the processes for obtaining increased yields of compounds of interest, faster and higher throughput screening methods for various host cell systems are still needed.

To date several methods are known that enable screening for increased production yield. Many approaches based on the mode-of-action of a specific protein, like enzyme-substrate interactions, protein-protein, or protein-ligand binding, are known. However, screening methods which are based on enzyme-substrate interactions are limited to substrate availability for a specific enzyme in the screening approach and are not applicable to non-catalytic proteins. Screening methods based on protein-protein or protein-ligand binding are also restricted to the specific binding partner. The screened variants obtained from such methods need to be locally separated or further transferred to a defined compartment.

Accordingly, such screening approaches are limited in the total number of variants that must be tested in these screening methods. Automation of the screening methods is used to bridge this gap, but this requires high investments and increases the complexity of the screening approach.

Other screening methods which are based on specific reporter system that are fused or coupled to a protein of interest, are also used. However, very often the presence of the reporter system does not correlate with the expression of the proteins of interest. In the worst case the utilized reporter system can fully abolish expression of a protein of interest.

Therefore, there is a need for simplified, high throughput screening methods for increased production of products, such as proteins, which screening methods could be utilized in various types of cellular expression systems, having high versatility and reliability, without the need for complex automation equipment. There is also a need for screening methods for increased production of products, such as proteins, in various cells, which method would not be restricted by the host cell selected but could be applied to many different types of host cells.

### SUMMARY

The appended claims define the scope of protection. Any examples and technical descriptions of apparatuses, products and/or methods in the description and/or drawings not covered by the claims are presented not as embodiments of the invention but as background art or examples useful for understanding the invention.

According to a first example aspect there is provided a screening method for cells having increased production of a thiol-containing compound, the method comprising the steps of:
(i) providing cells producing the thiol-containing compound;
(ii) cultivating the cells separately from each other in the presence of a sulfhydryl reagent; and
(iii) selecting cells exhibiting increased progression of cell cycle when compared to the rest of the cultivated cells, thereby obtaining a subgroup comprising cells having increased production of the thiol-containing compound.

The inventors have surprisingly found, and shown in the examples below, that a sulfhydryl reagent can be used to selectively screen for cells having increased production of one or more thiol-containing compounds. The beneficial cells having increased production of the thiol-containing compound can be selected and separated from the non-beneficial cells based on increased progression of cell cycle of the beneficial cells. Beneficial mutations improving the production of the thiol-containing compounds, such as proteins, of the cell, can be detected from these beneficial cells when culturing the cells under selection pressure caused by the sulfhydryl reagent. The beneficial mutations are further improving production of the thiol-containing compound, which can be detected in the current screening method as further increased progression of cell cycle and/or further induced thiol-containing compound production, when compared to a host cell without said beneficial mutations. Therefore, it was also found that a sulfhydryl reagent can be used to selectively screen for cell genotypes having increased production of thiol-containing compounds in general.

The principle of the screening is based on the finding that the higher the amount of the thiol-containing compound(s) expressed by the cell is, the faster is the used sulfhydryl reagent removed by conversion from the culture environment of the cell, and therefore, the faster the cell may overcome the sulfhydryl reagent-inhibited progression of cell cycle and continue the cell cycle. With the use of a sulfhydryl reagent which is a thiol-oxidizing agent, the cells used in the current method, such as a variant cell library, can be directly screened for higher thiol-containing compound production. The disclosed screening method is neither dependent on substrate interactions nor protein or ligand binding. Furthermore, the disclosed screening method approach does not have a negative influence on the expression level of the thiol- containing compound, such as a protein of interest, in the selected cells.

The disclosed screening method is highly versatile and can be applied to screening of all thiol-containing compounds, such as proteins (including, but not being limited to, non-catalytical proteins), metabolites, peptides and even free amino acids. Thus, the current method of the first aspect is beneficial, as the method is adjustable to many different host cell lines, and thiol-containing compounds to be produced. The current method of the first aspect is beneficial, as it allows screening for increased target compound, such as target protein production. The current method of the first aspect is beneficial, as it allows screening for increased production of proteins comprising cysteine residues. The current method is also beneficial, as it can be used to recognize beneficial mutations in the host cell genome, improving protein production (including protein expression and secretion) and/or protein stability in general. The current method is beneficial, as it can significantly reduce the amount of host cell genes to be investigated for beneficial effects on the protein production and/or stability. The current method is not limited by the total number of mutants to be tested in the procedure, the variant cell library size thereby being fully scalable. Different non-binding example aspects and embodiments have been illustrated in the foregoing. The embodiments in the foregoing are used merely to explain selected aspects or steps that may be utilized in different implementations. Some embodiments may be presented only with reference to certain example aspects. It should be appreciated that corresponding embodiments may apply to other example aspects as well.

### BRIEF DESCRIPTION OF THE FIGURES

Some example embodiments will be described with reference to the accompanying figures, in which:

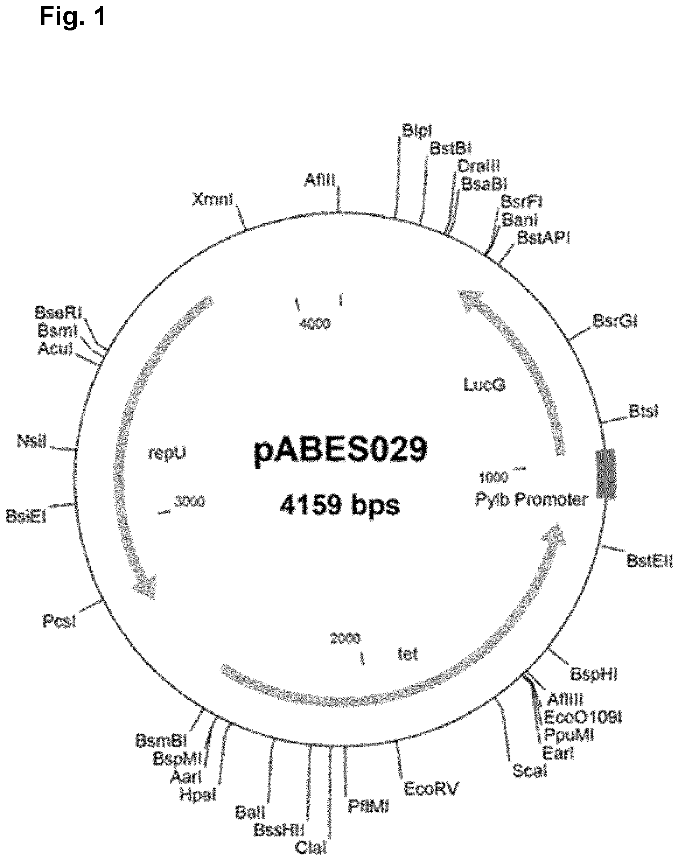

Fig. 1 shows a plasmid pABES029 comprising a synthetic *Gaussia princeps* luciferase gene (IucG; Accession: AY015993) under control of an Pylb-promoter within a pUB110 plasmid backbone.

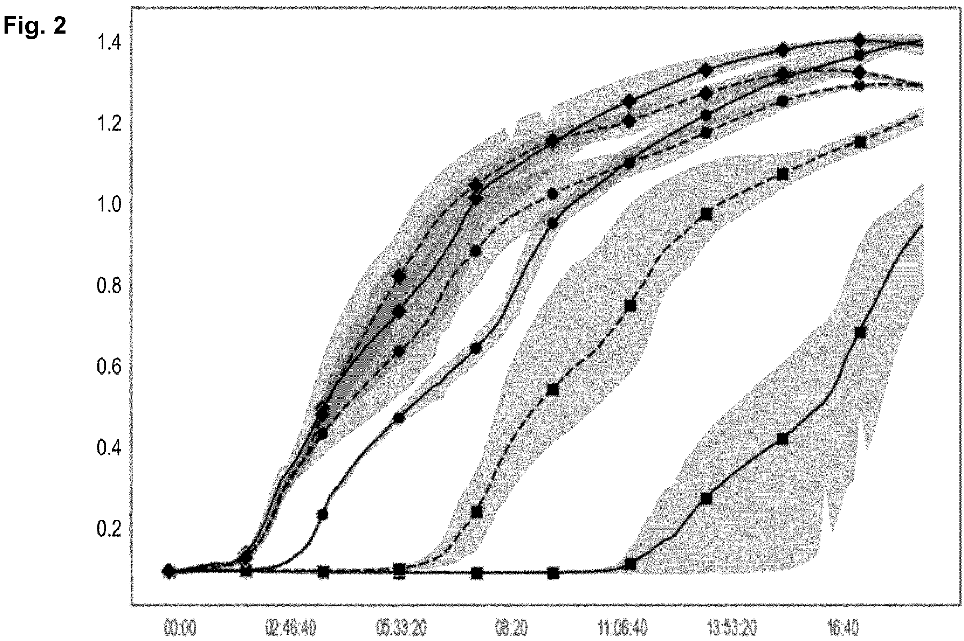

Fig. 2 shows growth inhibition (i.e. cell division inhibition) curves of *Bacillus subtilis* DB430 *ΔlipA* strain cells, treated with various diamide concentrations, measured as O.D. ₆₀₀ (Optical density at 600nm) values. The growth curves represent the host cell strain *Bacillus subtilis* DB30 Δ*lipA* (solid lines) and *Bacillus subtilis* DB30 Δ*lipA* containing the pABES029 expression plasmid expressing luciferase (dashed lines), cultured at +37 °C in the presence of 0 mM (◆), 0.1 mM (•), and 0.5 mM (■) of diamide. The duration of the culture period is indicated in the x-axis (time indicated as h:min:sec), whereas the O.D.₆₀₀ values are indicated in the y-axis. Grey areas surrounding the growth curves represent 95 % confidence interval of the measured O.D.₆₀₀ values.

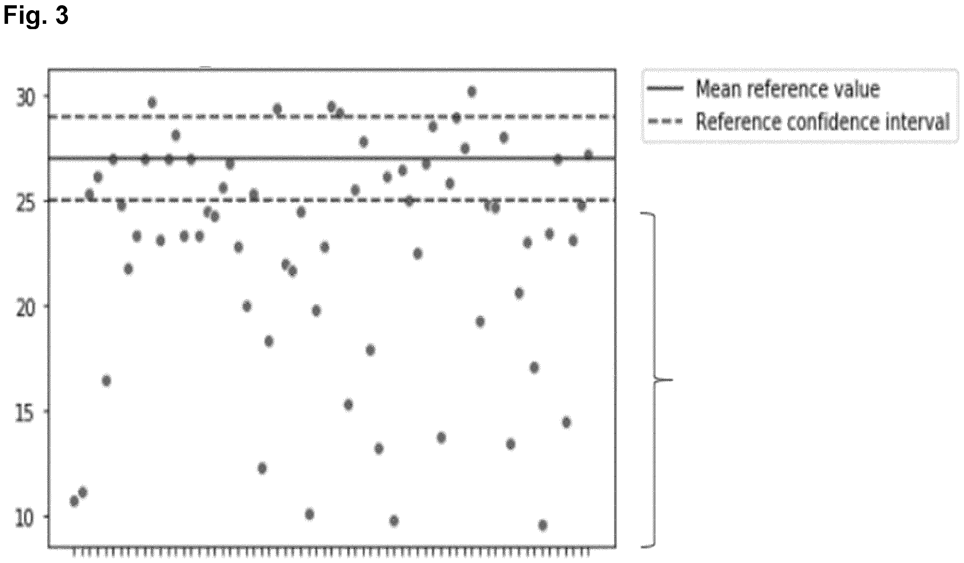

Fig. 3 shows data received from a kinetic diamide growth assay, performed with *Bacillus subtilis* DB430 *ΔlipA* variant cell library, containing the pABES029 expression plasmid and random transposon mutagenesis. The subgroup of cells for the kinetic diamide growth assay was selected from cells cultivated on LB agar plates containing 200 µM diamide. The DB430 *ΔlipA* cells containing the pABES029 expression plasmid, without transposon mutagenesis were used as a reference strain. Samples of the cell genotypes were inoculated from overnight cultures in ratio 1:20 to new medium and cultured at 37 °C in the presence of 2 mM diamide, under constant shaking. The O.D.₆₀₀ values were measured in 10 min intervals during 30 h. The measured data was analyzed by a computational method and plotted in the Figure 3. The growth delay (hours) of the measured cells is shown in the y-axis. The standard growth delay indicating the mean growth delay of the reference strain is shown as a solid line in the figure, and an established confidence interval for the standard growth delay is indicated with two dashed lines on both sides of the standard growth delay. The individual mutant cell genotypes (dots) falling below the lower confidence interval at 25-hour growth delay (i.e. cells exhibiting increased progression of cell cycle) were selected, making up the second subgroup of mutant cell genotypes.

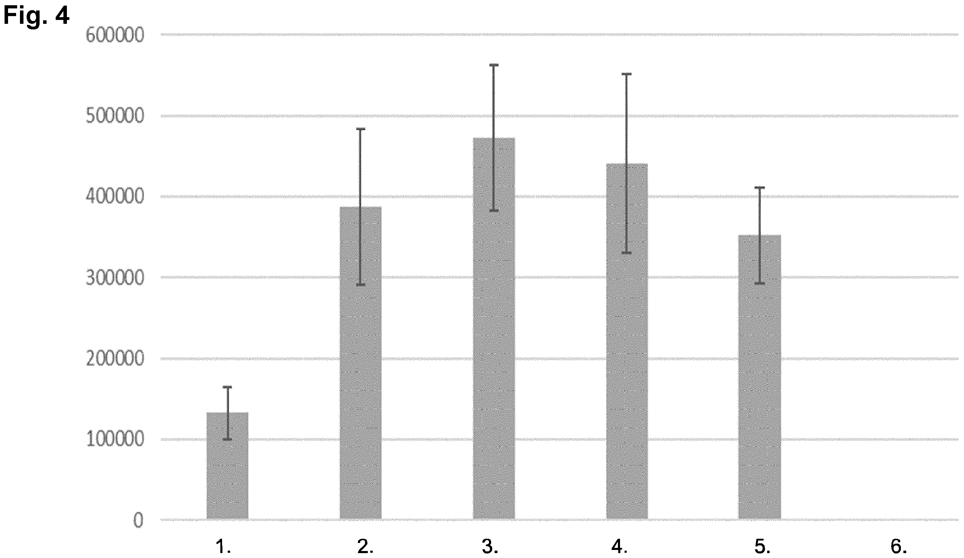

Fig. 4 shows *Gaussia princeps* luciferase expression quantification of four selected mutant cell genotypes comprising a seamless gene deletion of a target gene, to verify the identified mutant loci obtained by the screening method and a subsequent FPNI PCR locus identification. More specifically, sample 1 on the x-axis indicates the supernatant sample of the reference strain used, which was *B. subtilis* DB430 *ΔlipA* strain comprising the pABES029 luciferase expression vector. The samples 2-5 on the x-axis indicate the supernatant samples of the target gene deletion mutant strains of DB430 *ΔlipA* strain comprising the pABES029 luciferase expression vector, the sample numbers indicating the deleted genes as follows: sample No. 2. indicating Gene No. 2, sample No. 3. indicating Gene No. 3, sample No. 4. indicating Gene No. 4, and sample No. 5. indicating Gene No. 5. More details of the gene No. 2-5 disruptions are shown in the table 3. All the supernatant samples were collected after 6 h of cultivation and measured for luminescence at 480 nm in a microplate reader. The sample No. 6 on the x-axis indicates a blank measurement sample including pure LB medium, which was included to the measurement as a control.

### DETAILED DESCRIPTION

In the following description, like reference signs denote like elements or steps.

As used herein, the term "host cell" means any cell type that is provided and can be used in the current screening method. The term host cell can refer to a wild type cell comprising no induced mutations, or to a mutagenized host cell comprising either random or targeted mutations.

As used herein, the term "cell line" refers to continuous cell line obtained from a primary culture of said cells. The term cell line can refer to a eukaryotic or a prokaryotic cell line.

As used herein, the term "cell strain" refers to a population of cells arising from a cell line through selection.

As used herein, a "sulfhydryl reagent" refers to any chemical agent which reacts with a sulfhydryl (-SH) group. Therefore, sulfhydryl reagent does not refer to a chemical agent that must necessarily comprise sulfhydryl (-SH) group(s) *per se.* A sulfhydryl reagent may be a thiol-oxidizing agent, i.e. an agent which oxidizes free thiols and creates disulfide bonds between them. Examples of sulfhydryl reagents include, but are not limited to, tetramethylazodicarboxamide (diamide), 5,5'-dithiobis, N,N,N',N'-tetramethyldiazene-1,2-dicarboxamide, p-chloromercuribenzoate, and N-methylmaleimide. With "5,5'-dithiobis" is meant 2-nitrobenzoic acid, DTNB, or Ellman's reagent, which reacts with a free sulfhydryl group to yield a mixed disulfide and 2-nitro-5-thiobenzoic acid (TNB). N,N,N',N'-tetramethyldiazene-1,2-dicarboxamide is also a sulfhydryl reagent which oxidizes sulfhydryl groups to disulfide form. It is a radiation-sensitizing agent of anoxic bacterial and mammalian cells. N-Methylmaleimide is an electron deficient olefin that acts as a thiol-blocking reagent. With p-chloromercuribenzoate is meant 4-(Hydroxymercuri)benzoic acid, which is an organic mercurial compound that reacts via mercury-sulfur affinity with sulfhydryl groups in peptides, proteins and other molecules.

As used herein "diamide", also known as tetramethylazodicarboxamide (N,N,N',N'-Tetramethylazodicarboxamide) or 1,1'-Azobis(N,N-dimethylformamide), is a reagent used in biochemistry for oxidation of free thiols and create disulfide bonds between them.

As used herein, the term "thiol" refers to a sulfhydryl (-SH) group and to a sulfanyl group. As used herein, the term "thiol-containing compound" refers to a compound, such as a polypeptide, a peptide, an enzyme, a metabolite, thiolalcohol, a free cysteine residue or an amino acid sequence comprising at least one cysteine residue, which comprises at least one thiol-group. A thiol group is bound to an alkyl or other organic substituent in a molecule R-SH (wherein R represents the alkyl or the other organic substituent).

As used herein, the term "cysteine" or "Cys" refers to the amino acid with the formula HOOC-CH(-NH₂)-CH₂-SH, comprising a thiol side chain. Cysteine is encoded by the codons UGU and UGC.

As used herein, with the term "cell cycle" is meant the series of events in a cell, leading to its division into two daughter cells, said events including copying its genetic material and organelles (concerning only eukaryotes), changes in metabolism, cellular growth in size, and finally the actual division into two daughter cells. As used herein, with the term "increased progression of cell cycle" is meant, at least one of these events occurs in a faster rate, thereby eventually resulting in the event of cell division also faster.

As used herein, the term "growth arrest" or "growth delay" refers to cell cycle arrest of cells, wherein the cell cycle of a cell in question is not progressing into cell duplication and division but is halted in a stopping point in the cell cycle. As used herein, the term "growth arrest" or "growth delay" may also refer to cells, wherein the cell cycle is not in a complete standstill, but progressing in such a slow speed, that the progression is considered insignificant.

As used herein, the term "disulfide bond" or "disulfide bridge" refers to a bond formed between the sulfur atoms of cysteine residues in a polypeptide or a protein. Disulfide bridges can be naturally occurring, or non-naturally occurring, and, for example, introduced by way of amino acid substitution(s).

As used herein, the term "variant cell library" or "mutant cell library" means a collection or pool of cells, wherein mutations have been introduced to different parts of the genome of the cells, such that the cells of the library collectively comprise mutation(s) in all or selected genes and/or in all or selected areas of non-coding DNA (e.g. regulatory regions, promoters). In effect variant cell library comprises a collection or pool of mutant cells in which different genes and/or DNA regions are (selectively or non-selectively) mutated, "knocked out", or altered in their transcriptional activity in different cells of the library. The altered transcriptional activity of genes can refer to reduced expression and/or activity of the genes and thus, the variant cell library may comprise a collection or pool of mutant cells in which different genes are also "knocked down". Similarly, the altered transcriptional activity of genes can also refer to increased expression and/or activity of the genes. The genes and/or noncoding regions of DNA can be mutated, i.e. the variant cell library can be created with any suitable method known in the art, including, but not being limited to, UV-radiation, mutagenic agents, PCR-based methods, DNA shuffling, CRISPR/Cas based methods, nuclease and recombinase based methods, homologous recombination, transposon-based methods, or through any other suitable DNA altering technology and/or epigenetic modifier technology.

As used herein, the term "cell genotype" means the complete set of genetic material comprised by the specific cell in question, which may or may not be the same as a cell genotype of another cell. The phrase "selecting cell genotypes" refers to selecting cells comprising its own individual genotype.

The term " stability" in context of amino acid sequence or protein stability, describes as a function of time the property of protein to withstand and/or function in conditions that are not optimal for the activity and function of the protein in question, such conditions being, for example, high temperature, pressure, high or low pH, radiation, a certain concentration of inorganic salt or an organic solvent, or a specific composition comprising e.g. proteases, chaotropic agents, stabilizers and/or surfactants.

As used herein, "production" in context of thiol-containing compound, such as a polypeptide, an enzyme, or a metabolite, includes any step involved in the expression of a thiol-containing compound in question in a selected cell, including, but not being limited to, transcription, translation, post-translational modification, biosynthesis and/or secretion out of the cell. As used herein, "protein production" or "production" in context of polypeptides and proteins, such as a thiol-containing compound which is a protein, includes any step involved in the expression of a polypeptide or a protein in a selected cell including, but not limited to, transcription, translation, post-translational modification, and secretion out of the cell. Protein production may be followed by harvesting, i.e. recovering, the host cells or the expressed product.

As used herein, the term "comprising" includes the broader meanings of "including", "containing", and "comprehending", as well as the narrower expressions "consisting of" and "consisting only of". The words "comprise", "include", and "contain" are each used as open-ended expressions with no intended exclusivity.

In an embodiment, the current screening method for cells having increased production of a thiol-containing compound, comprises the steps of:
(i) providing cells producing the thiol-containing compound;
(ii) cultivating the cells separately from each other in the presence of a sulfhydryl reagent;
(iii) selecting cells exhibiting increased progression of cell cycle when compared to the rest of the cultivated cells, thereby obtaining a subgroup comprising cells having increased production of the thiol-containing compound.

In an embodiment, the step (i) of the current screening method comprises providing cells producing a thiol-containing compound. In an embodiment, the type of cell provided at the step (i) is selected from a prokaryotic or a eukaryotic cell, preferably the cell is Gram positive or negative bacteria, Archaea, fungal cell, yeast cell, plant cell, insect cell or mammalian cell, more preferably the cell is selected from a subgroup consisting of *Actinoplanes, Agrobacterium, Bacillus, Brevibacillus, Clostridium, Enterococcus, Escherichia, Erwinia, Geobacillus, Haemophilus, Lactobacillus, Lactococcus, Lysinibacillus, Oceanobacillus, Paenibacillus, Proteus, Pseudomonas, Rhizobium, Staphylococcus, Streptococcus, Streptomyces, Thermoactinomyces, Xanthomonas, Acremonium, Hyprocrea, Aspergillus, Aureobasidium, Bjerkandera, Ceriporiopsis, Chrysosporium, Coprinus, Coriolus, Cryptococcus, Filibasidium, Fusarium, Humicola, Kluyveromyces, Magnaporthe, Mucor, Myceliophthora, Neocallimastix, Neurospora, Paecilomyces, Penicillium, Phanerochaete, Phlebia, Pichia, Piromyces, Pleurotus, Saccharomyces, Schizophyllum, Talaromyces, Thermoascus, Thielavia, Tolypocladium, Trametes,* and *Trichoderma.*

In an embodiment, the cell provided at the step (i) is a cell of a cell line. In an embodiment, the cell provided at the step (i) is a cell of a cell strain. In an embodiment, the cell provided at the step (i) is selected from a prokaryotic or a eukaryotic cell.

In an embodiment, the cell provided at the step (i) is a microorganism. In an embodiment, the cell provided at the step (i) is a bacterial cell. In an embodiment, the cell provided at the step (i) is a fungal cell. In an embodiment, the cell provided at the step (i) is an insect cell or a mammalian cell.

In an embodiment, the cell provided at the step (i) is a prokaryotic cell. In an embodiment, the cell provided at the step (i) is a prokaryotic cell of a cell line. In an embodiment, the cell provided at the step (i) is a prokaryotic cell of a cell strain, such as a bacterial cell of a cell strain. In a preferred embodiment, the cell provided at the step (i) is *B. subtilis* cell.

In an embodiment, the cell provided at the step (i) is a eukaryotic cell. In an embodiment, the cell provided at the step (i) is a eukaryotic cell of a cell line. In an embodiment, the cell provided at the step (i) is a eukaryotic cell of a cell strain, such as a fungal cell of a cell strain. In a preferred embodiment, the cell provided at the step (i) is a *Trichoderma* cell.

In an embodiment, the cell provided at the step (i) is preferably a cell which is able to secrete produced products selected from free amino acids, peptides, polypeptides, enzymes and metabolites, such as cells from the genus *Aspergillus, Trichoderma, Bacillus, Streptomyces, Pichia, Saccharomyces* and *Escherichia.* In an embodiment, the host cell is most preferably selected from a genus *Aspergillus, Trichoderma, Bacillus, Streptomyces, Pichia, Saccharomyces* and *Escherichia.*

In an embodiment, the screening method screens for cells having increased production of a thiol-containing compound, the thiol-containing compound consisting of one type of thiol-containing compound, or a mixture of two or more different thiol-containing compounds. In an embodiment, the screening method screens for cells having increased production of at least one thiol-containing compound. In an embodiment, the screening method screens for cells having increased production of protein(s) comprising thiols. In an embodiment, the thiols are comprised by cysteine residues. In an embodiment, the increased production of the thiol-containing compound is detected as the increased progression of cell cycle of the selected cells at the step (iii).

In an embodiment, the current screening method screens for increased thiol-containing protein secretion of the cells. In an embodiment, the screening method screens for increased cysteine-comprising protein secretion of cells. In an embodiment, the current screening method screens for increased thiol-containing metabolite production of the cells. In an embodiment, the current screening method screens for increased thiolalcohol production of the cells.

In an embodiment, the current screening method screens for genes having a beneficial effect on the production of the thiol-containing compound, such as a protein, of the cell. In an embodiment, the current screening method comprising the cultivation step (ii) and the selection step (iii) significantly reduces the number of genes to be assessed for their effect on thiol-containing compound production, when compared to the total amount of genes present in the host cell.

In an embodiment, the thiol-containing compound produced by the cells is selected from an amino acid sequence comprising at least one cysteine residue; a free cysteine residue; a thiol-containing metabolite; a thiolalcohol; or any combination thereof.

In an embodiment, the thiol-containing compound produced by the cells is an amino acid sequence comprising at least one cysteine residue. In an embodiment, the current screening method is carried out with cells expressing at least one amino acid sequence comprising at least one cysteine residue. In an embodiment, the cell is a host cell expressing an amino acid sequence comprising at least one cysteine residue. In a preferred embodiment, the cell is a host cell secreting an amino acid sequence comprising at least one cysteine residue outside the host cell. In an embodiment, the cell is a host cell producing at least free cysteine residues. In an embodiment, the cell is a host cell producing at least a thiol-containing metabolite. In an embodiment, the cell is a host cell producing at least thiolalcohol.

In an embodiment, the cell provided at the step (i) is a non-mutagenized host cell, meaning that the genome of the host cell is not actively mutagenized prior to its use in the current method. Therefore, the cell provided at the step (i) can be a cell of a cell line or a cell strain but does not contain any added mutations prior to its use in the current method. In another embodiment, the cell provided at the step (i) carries at least one additional mutation in its genome prior to its use in the current method, such as deletion, substitution or insertion, wherein preferably the at least one additional mutation does not affect the genetic elements of the cell encoding the thiol-containing compound(s). In an alternative embodiment, the cell carries at least one mutation in its genome prior to its use in the current method, such as deletion, substitution or insertion, wherein preferably the at least one mutation does not reduce the production of the thiol-containing compound(s) of the cells.

In an embodiment, the amount of the thiol-containing compound(s) which is secreted from the cells is more important in the current method, than the intracellular amount of such compound. In such embodiments, the extracellularly secreted thiol-containing compound(s) and the (extracellularly) provided sulfhydryl reagent have better access to each other and thus, react faster, when compared to the time it takes for sulfhydryl reagent to enter the cell and react intracellularly. In some embodiments though, the increased progression of cell cycle according to the current screening method is observed due to intracellular sulfhydryl reagent conversion.

In an embodiment, the cells provided at the step (i) comprise cells producing sufficient amount of the thiol-containing compound(s), for the increased progression of cell cycle to be detected under the sulfhydryl reagent pressure. In an embodiment, the cells provided at the step (i) comprise cells secreting sufficient amount of the thiol-containing compound(s), for the increased progression of cell cycle to be detected under the sulfhydryl reagent pressure. The secreted thiol-containing compound(s) is/are secreted out of the cell, via active transport out of cell. The secreted thiol-containing compounds include the thiol-containing compounds which do not remain associated to the cell after the secretion. The secreted thiol-containing compounds include also the membrane-associated and/or the cell wall -associated thiol-containing compounds, such as amino acid sequences, which are either integral amino acid sequences spanning the cell membrane/wall and associated or attached to the membrane/wall of a cell, or peripheral amino acid sequences outside the cell, associated or attached to the membrane/wall of a cell and/or to other components which are attached to the cell membrane/wall.

In an embodiment, the thiol-containing compound is an amino acid sequence comprising at least one cysteine residue, and it is provided in the cells of the step (i) by:
(i.a) introducing into the cells genetic elements encoding an amino acid sequence comprising at least one, preferably at least 3, more preferably at least 5, even more preferably at least 10 cysteine residues, or
(i.b) providing the cells comprising genetic elements encoding an amino acid sequence comprising at least one, preferably at least 3, more preferably at least 5, even more preferably at least 10 cysteine residues.

Both, the number of cysteine residues in the amino acid sequence comprising at least one cysteine residue, expressed by the cells provided at the step (i), as well as the translated amount of the amino acid sequence comprising at least one cysteine residue produced by the cells, affects the duration of which the cells exhibit reduced progression of cell cycle and/or remain under the sulfhydryl reagent induced growth arrest. Therefore, if an exemplary cell candidate expresses the amino acid sequence comprising at least one cysteine residue wherein the number of cysteines is only one, the duration of the growth arrest of such cell may be shortened, if the total secreted amount of the amino acid sequence comprising at least one cysteine residue is high. What is described for the step (i) applies also for the steps (i.a) and (i.b). Thus, the steps (i) and (i.a), or (i) and (i.b), may be used interchangeably. In an embodiment, the step (i) comprises providing cells either by introducing into the cells genetic elements encoding an amino acid sequence comprising at least one cysteine residue, or by providing the cells comprising genetic elements encoding an amino acid sequence comprising at least one cysteine residue.

In an embodiment, the current screening method comprises the steps of:
(i) providing cells by:
   - introducing into a host cell genetic elements encoding an amino acid sequence comprising at least one cysteine residue, or
   - providing a host cell comprising genetic elements encoding an amino acid sequence comprising at least one cysteine residue;
(ii) cultivating the cells separately from each other in the presence of a sulfhydryl reagent;
(iii) selecting cells exhibiting increased progression of cell cycle when compared to the rest of the cultivated cells, thereby obtaining a subgroup comprising cells having increased production of the thiol-containing compound, which is the amino acid sequence comprising at least one cysteine residue.

In an embodiment, the amino acid sequence comprising at least one cysteine residue is provided in the cells of the step (i) with the alternative (i.a), and wherein less than 2 %, preferably less than 1 % of the amino acids comprised by secreted proteins of the cells are cysteines prior to providing said amino acid sequence comprising at least one cysteine residue into the cells.

In an embodiment, the cells used in the current method prior to providing the cells at the step (i) have a very low amount of translated amino acid sequences comprising cysteine (Cys) residues, such as 2 % or less, preferably 1.5 % or less, more preferably 1 % or less, or even more preferably 0.5 % or less of the amino acids comprised by translated amino acid sequences by the cells are cysteines.

In some embodiments, the cell used in the current method prior to providing it at the step (i) translates amino acid sequences comprising Cys-residues, but only very low amount of these Cys-containing amino acid sequences are secreted outside the cell. Therefore, in an embodiment, a very low amount, such as 2 % or less, preferably 1.5 % or less, more preferably 1 % or less, even more preferably 0.5 % or less of the amino acids comprised by secreted amino acid sequences from the cell are cysteines prior to providing said cell at the step (i).

In an embodiment, 2 % or less, preferably 1.5 % or less, more preferably 1 % or less, even more preferably 0.5 % or less of the amino acids comprised by the signal-peptide comprising proteins of the cell are cysteines prior to providing said cell at the step (i). In an embodiment, 0.01 % - 2 %, preferably 0.01 % - 1 % of the amino acids comprised by produced proteins of the cell are cysteines prior to providing said cell at the step (i).

Therefore, in an embodiment, wherein the amount of secreted amino acid sequences comprising cysteine (Cys) residues from the cell is very low, it is beneficial to introduce into the cell genetic elements encoding an amino acid sequence comprising at least one cysteine residue, prior to providing said cell at the step (i).

In an embodiment, the genetic elements of the cells provided at the step (i) encode an amino acid sequence comprising at least 3, preferably at least 5, more preferably at least 10, even more preferably at least 15 cysteine residues.

In an embodiment, the genetic elements of the cells provided at the step (i) encode an amino acid sequence comprising 1, 2, 3, 4, 5, 6, 7, 8, 9, 10, 11, 12, 13, 14 or 15 cysteine residues. In an embodiment, the genetic elements of the cells provided at the step (i) encode an amino acid sequence comprising 5 cysteine residues. In an embodiment, the genetic elements of the cells provided at the step (i) encode an amino acid sequence comprising 10 cysteine residues. In an embodiment, the genetic elements of the cells provided at the step (i) encode an amino acid sequence comprising 11 cysteine residues. The embodiments wherein the genetic elements of the cells provided at the step (i) encode an amino acid sequence comprising more than 1 cysteine residues are beneficial, as higher content of cysteines in the produced amino acid sequences shortens the duration of the sulfhydryl reagent-induced reduced progression of cell cycle and/or growth arrest of the cells, and thereby increases the screening sensitivity of the current method.

In an embodiment, it is expected that no further increase in cell cycle progression and/or reduction in the growth arrest during the cultivation of the step (ii) can be detected, when the genetic elements of the cells provided at the step (i) encode an amino acid sequence comprising more than 30, or more than 50 cysteine residues. Therefore, in an embodiment, the genetic elements of the cells provided at the step (i) encode an amino acid sequence comprising 1 - 50, preferably 3 - 30, more preferably 5 - 30, even more preferably 10 - 30, cysteine residues.

In an embodiment, the method comprises providing the cells at the step (i) by introducing into the cell genetic elements encoding the amino acid sequence comprising at least one, preferably at least 3, more preferably at least 5, even more preferably at least 10 cysteine residues.

In an embodiment, the method comprises providing the cells at the step (i) by introducing into the host cell genetic elements encoding an amino acid sequence comprising at least one cysteine residue, wherein less than 2 %, preferably less than 1 % of the amino acids comprised by secreted proteins of the cell are cysteines, prior to introduction of said genetic elements. In an embodiment, 0.01 % - 2 %, preferably 0.01 % - 1 % of the amino acids comprised by secreted proteins of the cell are cysteines prior to providing it at the step (i). In an exemplary embodiment, the cell is *Bacillus subtilis* 168 strain expressing approximately 296 signal peptide comprising amino acid sequences, from which 0.5 % comprise cysteines.

In an embodiment, the genetic elements encoding an amino acid sequence comprising at least one cysteine residue means genetic elements, such as DNA or RNA, which encode an amino acid sequence comprising at least one cysteine, preferably more than one cysteine. In an embodiment, the genetic elements encoding an amino acid sequence comprising at least one cysteine residue comprise translationally active mRNA and/or transcriptionally active DNA. In an embodiment, the genetic elements encoding an amino acid sequence comprising at least one cysteine residue are configured to express an amino acid sequence comprising at least one cysteine residue, meaning the genetic elements are able to express an amino acid sequence comprising at least one cysteine residue.

In an embodiment, providing the cells of the step (i) via the alternative (i.a) is beneficial, as according to this embodiment the current screening method allows selection of mutant cells which have the highest recombinant protein production, wherein the recombinant protein comprises the amino acid sequence comprising at least one cysteine residue. Moreover, according to this embodiment the current screening method allows screening for beneficial mutations affecting protein production and/or secretion of the mutant cells.

In an embodiment, the cells of the step (i) are provided via the alternative (i.b), by providing a cell already comprising the genetic elements encoding the amino acid sequence comprising at least one cysteine residue. In an embodiment, such cell is configured to secrete the amino acid sequence comprising at least one cysteine out of the cell. Therefore, in an embodiment, such cell expressing and secreting the cysteine-containing amino acid sequence, is provided in the step (i) without the need to introduce further genetic elements in said cell.

The embodiment wherein the cells of the step (i) are provided via the alternative (i.b) is beneficial in screening for beneficial mutations affecting protein production and/or secretion of the cells.

In an embodiment, the thiol-containing compound is configured to be secreted by the cells provided at the step (i).

In an embodiment, the thiol-containing compound is selected from an amino acid sequence comprising at least one cysteine residue, a free cysteine residue, a thiol-containing metabolite, or a thiolalcohol, and it is configured to be secreted by the cell. In an embodiment, the thiol-containing compound includes any combination of the amino acid sequence comprising at least one cysteine residue, free cysteine residues, and thiol-containing metabolites, and these are configured to be secreted by the cell. In an embodiment, the thiol-containing compound is the amino acid sequence comprising at least one cysteine residue, which is configured to be secreted by the cell.

In an embodiment, conditions for the cultivating at the step (ii) are selected from:
- a sulfhydryl reagent concentration effective to cause reduced progression of cell cycle or cell cycle arrest in the cultivated cells;
- cultivation takes place on a solid growth medium;
- cultivation takes place in a liquid growth medium; or
- any combinations thereof.

In an embodiment, the step (ii) of the method comprises the cells are cultivated in a sulfhydryl reagent concentration effective to cause reduced progression of cell cycle or cell cycle arrest in the cultivated cells. In an embodiment, in the presence of sufficient concentration of a sulfhydryl reagent, the cell growth is arrested.

In an embodiment, the step (ii) of the method comprises the cells are cultivated separately from each other. Cultivating the cells separately from each other allows the detection of individual cells exhibiting increased progression of cell cycle when compared to the rest of the cultivated cells. In an embodiment, the cultivation at the step (ii) takes place on a solid growth medium, the cultivation conditions being selected so that selecting individual cells or cell genotypes at the step (iii) is possible. In an embodiment, the provided cell is a microorganism, and the cultivation of the step (ii) takes place on a solid growth surface, such as an agar plate. In an embodiment, the cultivation at the step (ii) takes place on a solid growth medium, the cells being cultivated separately from each other as separate cell clone colonies.

In another embodiment, the cultivating of the step (ii) takes place in a liquid culture medium. In an embodiment, the cultivation at the step (ii) takes place in a liquid growth medium, the cultivation conditions being selected so that selecting individual cells or cell genotypes at the step (iii) is possible. In an exemplary embodiment, the cultivation of the step (ii) takes place in liquid culture medium of droplets, e.g., in a lipid droplet assay or alginate bead assay (Nanoliter reactor), wherein the cells are cultivated separately from each other. In an embodiment, the cells being cultivated separately means the cells are cultivated in separate culture medium droplets or beads. In an embodiment, the cells being cultivated separately means the cells are cultivated on separate culture dishes.

In an embodiment, as a result of the cultivation step (ii), some of the cells have started to continue the cell cycle and divide (i.e. have overcome the sulfhydryl reagent induced reduced progression of cell cycle and/or cell cycle arrest), allowing their selection in the step (iii). In an embodiment, the cultivation period of the cells at the step (ii) should be selected so, that only few cells have started to divide, i.e. have overcome the sulfhydryl reagent induced reduced progression of cell cycle and/or growth arrest, whereas most of the other cultured cells (reference cells) have not. If the cells are cultured for a period long enough for the reference cells to overcome the sulfhydryl reagent induced reduced progression of cell cycle and/or growth arrest and start dividing, the potentially beneficial cells exhibiting increased progression of cell cycle can no longer be detected over the other (reference) cells. In an embodiment, the reference cells at the cultivation step (ii), based on which the selection of the step (iii) is made, comprise the other cells not exhibiting progression of cell cycle, being cultivated at the step (ii), and/or the other cells of the subgroup. In an embodiment, the duration of the cultivation step (ii) is selected so, that 30 or fewer, 20 or fewer, 10 or fewer, or 5 or fewer of the cells exhibit increased progression of cell cycle, said cells being selected at the step (iii). In an embodiment, the step (ii) comprises cultivating the cells separately from each other in the presence of a sulfhydryl reagent until 30 or fewer, preferably 20 or fewer, more preferably 10 or fewer, even more preferably 5 or fewer of the cells exhibit increased progression of cell cycle.

In an embodiment, the cells provided at the step (i) comprise the genetic elements encoding an amino acid sequence comprising at least one cysteine residue, and the reference cells at the cultivation step (ii), based on which the selection of the step (iii) is made, comprise the cells without the genetic elements encoding an amino acid sequence comprising at least one cysteine residue. In such an embodiment, the duration of the cultivation step (ii) is selected so, that only some of the cells comprising genetic elements encoding an amino acid sequence comprising at least one cysteine residue have started exhibit increased progression of cell cycle, i.e. have overcome the sulfhydryl reagent induced reduced progression of cell cycle and/or growth arrest, whereas the reference cells without said genetic elements have not. In an embodiment, the duration of the cultivation step (ii) is selected so, that 30 or fewer, 20 or fewer, 10 or fewer, or 5 or fewer of the cells comprising genetic elements encoding an amino acid sequence comprising at least one cysteine residue exhibit increased progression of cell cycle, whereas the reference cells without said genetic elements do not. In an embodiment, the step (iii) comprises selecting cells exhibiting increased progression of cell cycle when compared to the cells without the genetic elements encoding an amino acid sequence comprising at least one cysteine residue, thereby obtaining a subgroup comprising cells having increased production of the thiol-containing compound.

In an embodiment, the sulfhydryl reagent is selected from diamide; 5,5'-dithiobis; DTNB; N,N,N',N'-tetramethyldiazene-1,2-dicarboxamide; p-chloromercuribenzoate; N-methylmaleimide; or any combination thereof, preferably the sulfhydryl reagent comprises diamide.

The sulfhydryl reagents are specific oxidants for thiol groups. The mode of action of the sulfhydryl reagents is to bind free thiols and create disulfide bonds between them. These interactions also occur on free cysteine residues of proteins (both intracellular and extracellular) and can be both inter- or intramolecular. As the sulfhydryl reagent in question reacts with free thiols, it is converted to other molecule(s) which does/do not interact with any further thiols. Once all sulfhydryl reagent has been sufficiently converted and cleared from the growth environment of the cell, the inhibitory effect on the cell cycle, or growth arrest of the cell is overcome, and the cellular growth is able to continue.

When the selected sulfhydryl reagent in question is diamide, the S-thiolations occur most commonly in low molecular weight thiols, such as bacillothiol, S-coA and cysteine residues. This type of thiolation is revertible. As the diamide molecule reacts with free thiols or degrades from heat and light, it is converted to hydrazine which does not interact with any further thiols. Once all diamide has been sufficiently cleared from the cell's growth environment, the growth arrest of the cell is overcome, and the cell cycle can continue.

In an embodiment, cultivating in the presence of a sulfhydryl reagent of the step (ii) means, the cultivation takes place in the presence of a sulfhydryl reagent concentration effective to cause delayed growth and division, and/or growth arrest in the cells in question. In a preferable embodiment, cultivating in the presence of a sulfhydryl reagent of the step (ii) means, the cultivation takes place in the presence of a sulfhydryl reagent concentration effective to cause growth arrest in the cells in question, thereby the cell cycle of the cells being treated with the sulfhydryl reagent being at a standstill.

In the presence of sufficient concentration of a sulfhydryl reagent such as diamide, the cell growth is arrested, i.e. the cell division is stopped, i.e. the cell cycle is stopped or at least significantly slowed down to the point where its progression is insignificant. In order for the growth arrest of the cells to occur, the sulfhydryl reagent concentration must be sufficiently high, the sufficiently high concentration being to some extent cell type dependent, as well as to some extent dependent on the exact sulfhydryl reagent used. The exact sulfhydryl reagent concentration which is effective to cause reduced progression of cell cycle and/or growth arrest in the cells is to some extent dependent on the host cell type selected. The exact sulfhydryl reagent concentration which is effective to cause decreased progression of cell cycle and/or growth arrest in the cells is to some extent also dependent on the exact sulfhydryl reagent selected. As the sufficient concentration of the sulfhydryl reagent is cell type and/or sulfhydryl reagent specific, small variations in the sufficient concentration of sulfhydryl reagent between different cell types can exist.

In an embodiment, the sulfhydryl reagent concentration used in liquid growth medium is higher than the concentration of the same sulfhydryl reagent on solid growth medium.

In an embodiment, cultivating in the presence of a sulfhydryl reagent of the step (ii) means, the cultivation takes place in the presence of at least 50 µM, preferably at least 100 µM sulfhydryl reagent concentration.

In an embodiment, the cultivation at the step (ii) takes place on solid growth medium, and in the presence of a sulfhydryl reagent concentration of 50 - 800 µM, preferably 100 - 400 µM. In an embodiment, a sulfhydryl reagent concentration of at least 50 µM, but at the most 800 µM is sufficient to cause reduced progression of cell cycle or growth arrest in the cells suitable for the current method on solid growth medium.

In an embodiment, the cultivation at the step (ii) takes place in liquid growth medium, and in the presence of a sulfhydryl reagent concentration of 50 - 5000 µM, preferably 500 - 4000 µM, more preferably 1000 - 2000 µM. In an embodiment, a sulfhydryl reagent concentration of at least 50 µM, but at the most 5000 µM is sufficient to cause reduced progression of cell cycle or growth arrest in the cells suitable for the current method in liquid growth medium.

In an embodiment, cultivating in the presence of a sulfhydryl reagent of the step (ii) means, the cultivation takes place in the presence of at least 50 µM diamide concentration.

In an embodiment, conditions for the cultivation at the step (ii) are selected from:
- a sulfhydryl reagent concentration effective to cause reduced progression of cell cycle and/or growth arrest in the cultured cells;
- duration of 10 - 80 h, preferably the duration is 20 - 60 h, more preferably the duration is 30 - 50 h, such as 40 h;
- diamide being used as the sulfhydryl reagent, preferably the diamide concentration is 50 - 800 µM, more preferably the diamide concentration is 100 - 400 µM, such as 200 µM;
- cultivation takes place on a solid growth medium; or
- any combinations thereof.

In an embodiment, the cultivation at the step (ii) takes place on a solid growth medium and in the presence of a diamide concentration of 50 - 800 µM, preferably the diamide concentration is 100 - 400 µM, more preferably the diamide concentration is 100 - 300 µM such as 200 µM. In an embodiment, the cultivation at the step (ii) takes place in the presence of a diamide concentration of 50 µM, 100 µM, 200 µM, 300 µM or 500 µM. In an embodiment, the cultivating at the step (ii) takes place on solid growth medium and in the presence of a 5,5'-dithiobis concentration of 50 - 800 µM. In an embodiment, the cultivating at the step (ii) takes place on solid growth medium and in the presence of a N,N,N',N'-tetramethyldiazene-1,2-dicarboxamide concentration of 50 - 800 µM. In an embodiment, the cultivating at the step (ii) takes place on solid growth medium and in the presence of a p-chloromercuribenzoate concentration of 50 - 800 µM. In an embodiment, the cultivation at the step (ii) takes place on solid growth medium and in the presence of a N-methylmaleimide concentration of 50 - 800 µM. In some embodiments, sulfhydryl reagent concentrations above 800 µM on solid growth medium may be used, if the selected host cell can withstand high sulfhydryl reagent concentrations, and the production of the thiol-containing compound(s), such as the amino acid sequence comprising at least one cysteine residue, of the cells is adequately high, to overcome the sulfhydryl reagent induced decreased progression of cell cycle and/or growth arrest.

In an embodiment, the cultivation at the step (ii) takes place in liquid growth medium and in the presence of a diamide concentration of 50 - 5000 µM, more preferably the diamide concentration is 500 - 4000 µM, even more preferably the diamide concentration is 1000 - 2000 µM, such as 2000 µM. In an embodiment, the cultivation at the step (ii) takes place in the presence of a diamide concentration of 50 µM, 500 µM, 1000 µM, 1500 µM or 2000 µM. In an embodiment, the cultivation at the step (ii) takes place in liquid growth medium and in the presence of a 5,5'-dithiobis concentration of 50 - 5000 µM. In an embodiment, the cultivation at the step (ii) takes place in liquid growth medium and in the presence of a N,N,N',N'-tetramethyldiazene-1,2-dicarboxamide concentration of 50 - 5000 µM. In an embodiment, the cultivation at the step (ii) takes place in liquid growth medium and in the presence of a p-chloromercuribenzoate concentration of 50 - 5000 µM. In an embodiment, the cultivation at the step (ii) takes place in liquid growth medium and in the presence of a N-methylmaleimide concentration of 50 - 5000 µM. In some embodiments, sulfhydryl reagent concentrations above 2000 µM or 5000 µM in liquid growth medium may be used, if the selected host cell can withstand high sulfhydryl reagent concentrations, and the production of the thiol-containing compound(s), such as the amino acid sequence comprising at least one cysteine residue, of the cells is adequately high, to overcome the sulfhydryl reagent induced decreased progression of cell cycle and/or growth arrest. In an embodiment, the cultivation at the step (ii) takes place in a liquid growth medium and cultivating in the presence of a sulfhydryl reagent means, the cultivation takes place in the presence of at least 50 µM, preferably at least 500 µM sulfhydryl reagent concentration.

In an embodiment, the cultivation at the step (ii) has a duration in which some of the cells have (re)started to divide, i.e. have overcome the sulfhydryl reagent induced reduced progression of cell cycle and/or growth arrest, whereas the majority of the other cells are not yet dividing, i.e. have not overcome the sulfhydryl reagent induced growth arrest.

In an embodiment, the cultivation at the step (ii) takes place in liquid or on solid growth medium and has a duration of 10 - 80 h, preferably the duration is 20 - 60 h, more preferably the duration is 30 - 50 h, such as about 40 h. In an embodiment, the step (ii) comprises cultivating the cells separately from each other in the presence of a sulfhydryl reagent until 30 or fewer, preferably 20 or fewer, more preferably 10 or fewer, even more preferably 5 or fewer of the cells exhibit increased progression of cell cycle, but not less than 10 h or longer than 80 h.

In an embodiment, the cultivation at the step (ii) takes place on solid growth medium in the presence of a diamide concentration of 50 - 800 µM for a duration of 10 - 80 h. In an embodiment, the cultivation at the step (iii) takes place on solid growth medium in the presence of a diamide concentration of 100 - 400 µM for a duration of 20 - 60 h.

In an embodiment, the cultivation at the step (ii) takes place in liquid growth medium in the presence of a diamide concentration of 50 - 5000 µM for a duration of 10 - 80 h. In an embodiment, the cultivation at the step (ii) takes place in liquid growth medium in the presence of a diamide concentration of 500 - 4000 µM for a duration of 20 - 60 h.

In an embodiment, the method further comprises:
(iv) cultivating in the presence of a sulfhydryl reagent the cells of the subgroup from the step (iii), thereby obtaining cell genotypes of the cells of the subgroup; and
(v) selecting cell genotypes exhibiting increased progression of cell cycle when compared to the rest of the cell genotypes of the subgroup, thereby obtaining a second subgroup comprising cells having increased production of the thiol-containing compound(s).

In an embodiment, cultivating in the step (iv) means, that each cell of the subgroup is cultivated separately from other cells of the subgroup. In an embodiment, cultivating in the step (iv) takes place in liquid growth medium or on solid growth medium. In an embodiment, cultivating in the step (iv) takes place on solid growth medium on an agar plate, or in liquid culture medium in multi-well plates or in culture vessel, or in any medium allowing the selection between cell genotypes at the step (v). For example, in an embodiment, the cells are cultivated in the step (iv) on a multi-well plate, each individual genotype being cultured in its own well, i.e. the genotypes are cultivated separately from each other.

In an embodiment, the amount of the thiol-containing compound(s) produced by the cell has an effect on the duration of the phase of reduced growth and/or growth arrest of the cells during the cultivation step (ii) and/or (iv). Accordingly, in an embodiment, the higher the amount of thiol residues in compounds produced by the cell, the shorter is the phase of reduced progression of cell cycle and/or growth arrest of the cells during the cultivation step (ii) and/or (iv). In an embodiment, the cultivation at the step (iv) has a duration in which some of the selected cells of the (first) subgroup from the step (iii) have started to divide, i.e. have overcome the sulfhydryl reagent induced reduced progression of cell cycle and/or growth arrest, whereas the majority of the other cells are not yet dividing, i.e. have not overcome the sulfhydryl reagent induced growth arrest.

In an embodiment, the cultivating at the step (iv) takes place in the presence of a sulfhydryl reagent concentration which is effective to cause reduced progression of cell cycle and/or growth arrest in the cells. Similarly, as in the cultivation step (ii), the exact sulfhydryl reagent concentration which is effective to cause growth arrest in the cells is to some extent dependent on the cell type selected, and/or the exact sulfhydryl reagent selected. In an embodiment, the cultivating at the step (ii) takes place on a solid growth medium and the cultivation at the step (iv) takes place in liquid growth medium, and thus, the cultivation at the step (iv) can take place in the presence of a higher sulfhydryl reagent concentration than the cultivation at the step (ii) of the process. In an alternative embodiment, the cultivation at the step (ii) takes place in liquid growth medium and the cultivation at the step (iv) takes place on solid growth medium. In a yet alternative embodiment, cultivation in the step (iv) is done as the cultivation in the step (ii). The suitable sulfhydryl reagents used at the step (ii), apply also for the sulfhydryl reagents used at the step (iv). The suitable concentrations disclosed for the sulfhydryl reagents used at the step (ii) on solid or in liquid growth media, apply also for the sulfhydryl reagents used at the step (iv) on solid or in liquid growth media, respectively. The suitable cultivation durations disclosed for the cells at the step (ii) on solid or in liquid growth media, apply also for the cells cultivated at the step (iv) on solid or in liquid growth media, respectively.

In an embodiment, the cultivation at the step (iv) takes place in liquid growth medium, the cultivation conditions being selected so that selecting separate genotypes of the cell subgroup obtained from the step (iii) is possible. In an exemplary embodiment, the step (iii) of the process comprises selecting cell genotypes by inoculating cell colonies from solid agar plates, and step (iv) of the process comprises cultivating said inoculated cell colonies as separate genotypes in liquid growth medium.

In an embodiment, as a result of the cultivation step (iv), separate cell genotypes of the cell subgroup obtained at the step (iii) are grown, allowing their selection in the step (v). In an embodiment, after the selected duration of the cultivation step (iv), only some of the separate genotypes of the cell subgroup from the step (iii) have continued the cell cycle i.e. have overcome the sulfhydryl reagent induced reduced progression of cell cycle and/or growth arrest, whereas most of the other cultured cells from the step (iv) (reference cells) have not. Similarly, as in the selection step (iii), if the cells are cultured for a period long enough for the reference cells to overcome the sulfhydryl reagent induced reduced progression of cell cycle and/or growth arrest and start dividing, the potentially beneficial cell genotypes exhibiting increased progression of cell cycle can no longer be detected over the other (reference) genotypes. Therefore, the cultivation period of the cells at the step (iv) must be adjusted so, that only few cells have started to divide, whereas the other (reference) cells have not.

In an embodiment, the reference cells at the cultivation step (iv), based on which the selection of the step (v) is made, comprise the other cells not exhibiting progression of cell cycle and being cultivated at the step (iv), and/or the other cells of the second subgroup. In such an embodiment, the duration of the cultivation step (iv) is selected as in the step (ii). In an embodiment, the step (iv) comprises cultivating the cells separately from each other in the presence of a sulfhydryl reagent until 30 or fewer, preferably 20 or fewer, more preferably 10 or fewer, even more preferably 5 or fewer of the cells exhibit increased progression of cell cycle.

In an embodiment, the cells provided at the step (i) comprise the genetic elements encoding an amino acid sequence comprising at least one cysteine residue, and the reference cells at the cultivation step (iv), based on which the selection of the step (v) is made, comprise the cells without the genetic elements encoding an amino acid sequence comprising at least one cysteine residue. In such an embodiment, the duration of the cultivation step (iv) is selected as in the step (ii). In an embodiment, the step (v) comprises selecting cells exhibiting increased progression of cell cycle when compared to the cells without the genetic elements encoding an amino acid sequence comprising at least one cysteine residue, thereby obtaining the second subgroup comprising cells having increased production of the thiol-containing compound(s).

In an embodiment, conditions for the cultivating at the step (iv) are selected from:
- a sulfhydryl reagent concentration effective to cause reduced progression of cell cycle and/or growth arrest in the cultured cells;
- duration of 10 - 80 h, preferably the duration is 20 - 60 h, more preferably the duration is 30 - 50 h, such as 40 h;
- diamide being used as the sulfhydryl reagent, preferably the diamide concentration is 50 - 5000 µM, more preferably the diamide concentration is 500 - 4000 µM, even more preferably the diamide concentration is 1000 - 2000 µM;
- cultivation takes place in liquid growth medium; or
- any combinations thereof.

In an embodiment, the screening method comprises two different cultivation and two different selection steps. In such an embodiment, the selected cells of the subgroup at the step (iii) are further cultivated in the cultivation step (iv), followed by the selection in the step (v). In an embodiment, the screening method comprises the two different cultivation steps (iii) and (iv), and the two different selection steps (iii) and (v).

In an embodiment, the screening method comprising said two different cultivation and two different selection steps is beneficial, as in the second subgroup the number of cell genotypes is generally further reduced from the number of cell genotypes comprised in the (first) subgroup from the step (iii). Therefore, the screening method comprising said two different cultivation and two different selection steps requires assessing protein production of fewer cells, than a screening method comprising only one cultivation and selection step. In an embodiment, the screening method comprising said two different cultivation and two different selection steps can reduce the number of cells to be assessed for protein production at least 20 %, preferably at least 40 % when compared to the screening method comprising only one cultivation and selection step. In an embodiment, the current screening method comprising the two cultivation steps (ii) and (iv), and the two selection steps (iii) and (v) can reduce the number of cell genotypes and genes to be assessed for their effect on protein production, when compared to a method comprising only one cultivation and selection step.

In an embodiment, the current screening method comprises more than two cultivation steps and more than two selection steps, such as three or four cultivation and selection steps.

In an embodiment, the amount of the amino acid sequence comprising at least one cysteine residue secreted by the cells of the (first) subgroup (from step (iii)) and/or the second subgroup (from step (v)); and/or the number of cysteine residues in the amino acid sequence comprising at least one cysteine residue secreted by the cells of the (first) subgroup and/or the second subgroup, correlates with a reduction in a duration of the growth arrest at the cultivation step (ii) and/or (iv).

In an embodiment, the translated amount of the amino acid sequence comprising at least one cysteine residue secreted by the cells of the (first) subgroup and/or the second subgroup, correlates positively with a reduction in a duration of the growth arrest at the cultivation step (ii) and/or (iv).

In an embodiment, the number of cysteine residues in the amino acid sequence comprising at least one cysteine residue secreted by the cells of the (first) subgroup and/or the second subgroup, correlates positively with a reduction in a duration of the growth arrest at the cultivation step (ii) and/or (iv).

In an embodiment, the selecting of cells exhibiting increased progression of cell cycle at the step (iii) and/or at the step (v) is based on:
- optically detectable phenotypic selection, preferably the selection comprises selecting cells which are optically detectable earlier when compared to the rest of the cells;
- a measurement of metabolic activity, or
- any combinations thereof.

For cell cycle and division to continue, the sulfhydryl reagent must be removed from the growth environment of the cells. In an embodiment, selected cells at the step (iii) and/or at the step (v) have increased progression of cell cycle and/or a shorter cell cycle arrest when compared to corresponding reference cells, as said cells require a shorter time period to remove the sulfhydryl reagent, such as diamide, from the growth environment of the cells and overcome its inhibitory effect on the cell cycle.

In an embodiment, the progression of cell cycle of the selected cells is increased when compared to corresponding reference cells, as said cells have an increased production of the thiol-containing compound(s) and hence, require a shorter time to remove the sulfhydryl reagent from the growth environment of the cells and continue the cell cycle. In an embodiment, the higher the amount of thiols in the thiol-containing compound(s) produced, the more thiol groups are present to react with a sulfhydryl reagent, and hence, the sooner the sulfhydryl reagent in question is converted in the growth environment of the cells. In an exemplary embodiment, the sulfhydryl reagent is diamide, and the thiol-containing compound produced by the cells is an amino acid sequence comprising at least one cysteine residue, and the higher the amount of said amino acid sequence is produced, the more cysteine residues are also present to react with diamide, thus resulting in diamide being converted to hydrazine faster and being removed from the growth environment of the cells.

In an embodiment, the selection of the step (iii) and/or (v) takes place immediately after the cultivation of the step (ii) and/or (iv), respectively. In an embodiment, selecting at the step (iii) and/or at the step (v) means, that more than one cell genotype is selected based on the cell's ability to overcome the sulfhydryl reagent-induced reduced progression of cell cycle and/or growth arrest. In an alternative embodiment, selecting at the step (iii) and/or at the step (v) means, that only one cell genotype is selected, in case no other cells can continue the cell cycle.

In an embodiment, the selection of the cell genotypes exhibiting increased progression of cell cycle at the step (iii) and/or the step (v) is based on optical detection of the cells overcoming the sulfhydryl reagent induced cell cycle arrest, and thus, restart of the cell cycle. In an embodiment, the selection of the cells and/or cell genotypes at the step (iii) and/or the step (v) is based on optically detectable phenotypic selection, preferably selection comprises selecting cell genotypes which are optically detectable earlier when compared to the cultivated reference cells. In an embodiment, the selection at the step (iii) and/or at the step (v) is done before the genotypes of the corresponding reference cell are optically detectable. In an embodiment, the selection of cells exhibiting increased progression of cell cycle at the step (iii) and/or at the step (v) means, the cells which continue the cell cycle first and start to divide before the corresponding reference cells, are selected. For example, the optical detection is done by detecting cell colonies on agar plates, and the selection of the cell colonies at the step (iii) and/or at the step (v) is done when only few colonies, such as 30 or fewer, preferably 20 or fewer, more preferably 10 or fewer, even more preferably 5 or fewer, are detectable on the agar plate. According to another example, the optical detection at the step (iii) and/or at the step (v) is done by detecting cell cycle progression in cells grown in liquid growth medium in a lipid droplet assay and the selection of the cells exhibiting increased progression of cell cycle is done based on measurement of optical density of the cell droplets. In an embodiment, the selection of the cells/ cell genotypes at the step (iii) and/or the step (v) is done with an optical measurement, such as optical density measurement.

When cells are grown on a solid surface (such as agar plate) in colonies, smaller satellite colonies are likelier to form near to the colonies which have first depleted sulfhydryl reagent from the surrounding environment on the growth surface. In an embodiment, the cultivation at the step (ii) and/or (iv) takes place on a solid growth medium and the selection at the step (iv) and/or (v) comprises selecting cell genotypes having peripheral satellite colonies.

In an embodiment, the optically detectable phenotypic selection can be based on, but is not limited to, measurements of cell colony formation, optical measurement of the cell culture medium factors such as density/turbidity, optical measurement of cell volume or cell mass (e.g., microscopy methods), visualized marker gene expression, or optical detection of consumption or production of substances, (i.e. detection of metabolic activity). In an embodiment, the possible methods for the optically detectable phenotypic selection include, but are not limited to, visual detection, measurement of optical density, light-, fluorescence-, high-resolution microscopy and flow cytometry-based methods.

Substantial changes occur in the metabolic activity of a cell once cell cycle of a quiescent cell progresses to cell division. In an embodiment, the selection of the cells and/or cell genotypes at the step (iii) and/or the step (v) is based on a measurement of metabolic activity. In an embodiment, as a measurement of metabolic activity, for example, measurements of oxygen consumption, CO₂ production or proton excretion (extracellular acidification rate) of the cell, reflecting activity of glycolysis pathway, can be used.

In some embodiments, the step (v) is the second selection step (i.e. second screening round) of the process, and the obtained second subgroup comprises cell genotypes exhibiting further increased progression of cell cycle and/or further shortened average growth arrest, when compared to the (first) cell subgroup obtained at the step (iii).

In an embodiment,
- the subgroup obtained at the step (iii) comprises cells having increased production of the thiol-containing compound(s) compared to the rest of the cultivated cells of the step (ii), and/or
- the second subgroup obtained at the step (v) comprises cells having increased production of the thiol-containing compound(s) compared to the rest of the cultivated cells of the step (iv).

In an embodiment, the selected cells obtained at the step (iii) and/or at the step (v) include cell genotypes with beneficial mutations inducing the expression and/or stability of proteins produced by said genotypes. In an embodiment, the subgroup obtained at the step (iii) comprises cells having increased production of the thiol-containing compound(s) compared to the reference cells cultivated in the step (ii). In a preferable embodiment, both the subgroup obtained at the step (iii) and the second subgroup obtained at the step (v) comprise cells having increased production of the thiol-containing compound(s) compared to the reference cells, cultivated in the step (ii) and (iv), respectively. In a preferable embodiment, both the subgroup obtained at the step (iii) and the second subgroup obtained at the step (v) comprise cells having increased protein production compared to the reference cells, cultivated in the step (ii) and (iv), respectively.

In an embodiment, the current screening method for cells having increased production of thiol-containing compounds, is a screening method for increased protein production of cells.

In an embodiment, the cells of the step (i) comprise a variant cell library, wherein the variant cell library is preferably obtained by:
- introducing into the cells random mutagenesis;
- introducing into the cells targeted mutagenesis;
- introducing into the cells targeted mutagenesis thereby obtaining cell variants of a gene of interest; or
- any combinations thereof.

The terms "variant cell" and "mutant cell" can be used interchangeably. In an embodiment, the mutagenesis of the cells is generated through any suitable method known for a skilled person, which results in the variant cell library comprising cells producing the thiol-containing compound(s).

In an embodiment, the mutagenesis is generated through randomized or targeted mutagenesis. In an embodiment, the mutagenesis is generated through randomized mutation, wherein mutations are inserted into random locations in the genome of the cell. For example, the mutagenesis is generated through randomized mutation obtained with transposon mutagenesis, thereby obtaining the variant cell library comprising random mutations. In an embodiment, the mutagenesis is generated through introduction of targeted mutagenesis into the cells, thereby obtaining cell variants of a gene of interest. In an embodiment, the mutagenesis is generated through targeted mutations, wherein mutations are inserted into pre-determined locations in the genome of the cells, resulting in the variant cell library. In an embodiment, the mutagenesis is generated through targeted mutations, wherein mutations are inserted into pre-determined locations in the genome of the cells by homologous recombination.

In an embodiment, the mutagenesis is generated through physical mutagenesis such as heat induced mutagenesis, or radiation induced mutagenesis such as UV-radiation, thereby obtaining the variant cell library. In an embodiment, the mutagenesis is generated through chemical mutagenesis such as mutagenic agents including, but not being limited to, base analogs, intercalating agents, metal ions and alkylating agents, thereby obtaining the variant cell library. In an embodiment, the mutagenesis is generated through biological mutagenesis such as transposons and viruses, thereby obtaining the variant cell library. In an embodiment, the mutagenesis is generated through random TnYLB-1 transposon-based mutagenesis. In an embodiment, the mutagenesis is generated through UV-radiation, mutagenic agent, PCR-based methods, DNA shuffling, homologous recombination, transposon-based methods, or through any other suitable DNA altering technology.

In an embodiment, the step (i) of the method comprises providing the cells by introducing into the cells mutagenesis, thereby obtaining a variant cell library comprising cells producing the thiol-containing compound(s). In an embodiment, the variant cell library comprises a collection of host cells wherein mutations have been introduced to different parts of the genome in different cells. In an embodiment, each cell of the variant cell library has at least one mutation in its genome. In an embodiment, each cell of the variant cell library has a different mutation in its genome than the mutations comprised by the other cells. In an embodiment, the variant cell library comprises a collection of host cells, wherein the mutation(s) affect the host cell's ability to produce the thiol-containing compound(s), such as cysteine-containing compound(s), depending on the integration site of the mutation.

In an embodiment, some of the mutations comprised by the cells of the variant cell library have a beneficial effect on the thiol-containing compound production of said cells. In an embodiment, some of the mutations comprised by the cells of the variant cell library have a beneficial effect on the protein production of said cell, the protein expression, and/or secretion and/or stability being increased. In an embodiment, the mutations comprised by the cells of the variant cell library having a beneficial effect on the protein stability includes, but is not limited to, mutations affecting protein folding and/or mutations decreasing expression of proteases.

In an embodiment, some of the other mutations comprised by the cells of the variant cell library have an unbeneficial effect on the thiol-containing compound expression, or no effect on the thiol-containing compound expression of the cells in question. In an embodiment, some of the other mutations comprised by the cells of the variant cell library have an unbeneficial effect on the protein expression, or no effect on the protein expression of the cells in question. In an embodiment wherein the mutations comprised by the cells of the variant cell library have unbeneficial effects on the protein expression of cells in question, the mutations include, but are not limited to, lethal mutations, mutations inhibiting protein production, mutations reducing cell viability, and/or mutations inhibiting cell cycle.

In an embodiment, the cells of the variant cell library express the thiol containing compound(s). In an embodiment, the cells of the variant cell library express the amino acid sequence comprising at least one cysteine residue. In an embodiment, the cells of the variant cell library comprise mutations inducing the expression and/or stability of the thiol containing compound, such as the amino acid sequence comprising at least one cysteine residue. In an embodiment, the cells of the variant cell library comprise mutations inducing the expression and/or stability of amino acid sequences and proteins in general.

In an embodiment, the step (i) is followed by a step (i.2), wherein mutagenesis is introduced into the provided cells (i.e. host cells), thereby obtaining the variant cell library.

In the following, the cultivation step (ii) which comprises cultivating separately in the presence of a sulfhydryl reagent the cell genotypes of the host cell and the cell genotypes of the variant cell library is marked as a step (ii.1), and the selection step (iii) which comprises selecting the cell genotypes of the variant cell library exhibiting increased progression of cell cycle when compared to the cell genotypes of the host cell, is marked as a step (iii.1). Nevertheless, what is described for the steps (ii) and (iii), applies also for the steps (ii.1) and (iii.1), respectively. Thus, the steps (ii) and (ii.1); and the steps (iii) and (iii.1) may be used interchangeably.

In an embodiment, the current screening method comprises the steps of:
(i) providing host cells;
(i.2) introducing into the host cells mutagenesis, thereby obtaining a variant cell library;
(ii.1) cultivating separately, in the presence of a sulfhydryl reagent
   - the host cells provided at the step (i), thereby obtaining cell genotypes of the host cell, and
   - the variant cell library, thereby obtaining cell genotypes of the variant cell library; and
(iii.1) selecting cell genotypes of the variant cell library exhibiting increased progression of cell cycle when compared to the cell genotypes of the host cell, thereby obtaining a first set of mutant cell genotypes which is the subgroup comprising cells having increased production of the thiol-containing compound(s).

In an embodiment, some of the mutations comprised by the cells of the variant cell library have an effect on the thiol-containing compound of said cells, the effect of the mutation being selected from altered translation, altered secretion, and/or altered stability of the thiol-containing compound. In an embodiment, some of the mutations comprised by the cells of the variant cell library have an effect on the protein secretome of said cells, the effect of the mutation being selected from altered protein translation, altered protein secretion, and/or altered protein stability. In an embodiment, the mutations introduced to the cells at the mutagenesis of the step (i.2) comprise beneficial and unbeneficial mutations concerning protein expression of the cells. However, the current screening method is configured to specifically screen for the mutant cell genotypes comprising beneficial mutations which increase the production of the thiol-containing compound(s) in the mutant cells. Therefore, in an embodiment, the current screening method is a screening method also for beneficial mutations, affecting the protein secretome of the mutant cells of the variant cell library. In an embodiment, the screening method screens for beneficial mutations, affecting the protein secretome of the mutant cells, when compared to the other host cells and/or other cells of the variant library. In an embodiment, the screening method screens for beneficial mutations affecting the production of the thiol-containing compound, such as an amino acid sequence comprising at least one cysteine residue, of the mutant cells, when compared to the other host cells and/or other cells of the variant library.

In an embodiment, the method comprises screening for mutant cell genotypes, wherein a mutation introduced at the mutagenesis of the step (i.2) increases the protein production of said mutant cell genotypes. In an embodiment, the increased protein production comprises increased protein expression and/or protein secretion of said mutant cell genotypes. In an embodiment, the method comprises screening for mutant cell genotypes wherein a mutation or mutations introduced at the mutagenesis of the step (i.2), increases the protein stability of said mutant cell genotypes. In an embodiment, the increased protein production and/or stability of said mutant cell genotypes is detected as the increased progression of cell cycle and/or shortened growth arrest duration, and/or as increased protein expression of said mutant cell genotypes.

In an embodiment, the cultivating at the step (ii.1) takes place in the presence of a sulfhydryl reagent concentration which is effective to cause decreased progression of cell cycle and/or growth arrest in the provided host cells, as well as in the cells of the variant cell library. In an embodiment, the cultivating at the step (ii.1) comprises the same conditions as cultivating at the step (ii). In an embodiment, the reference cells at the cultivation step (ii.1), based on which the selection of the step (iii.1) is made, comprise the cell genotypes of the host cell cultivated at the step (ii.1). In an embodiment, the cultivation of the step (ii.1) is done according to the cultivation step (ii), and additionally, the host cells (i.e. the reference cells) are cultivated in parallel separately, in addition to the cells of the variant cell library. In an embodiment, the selection step (iii.1) is done according to the selection step (iii), and by using the cell genotypes of the host cell as the reference cells.

In an embodiment, the cultivating at the step (ii.1) has a duration in which the some of the cells of the variant cell library have started to exhibit increased progression of cell cycle, i.e. have overcome the sulfhydryl reagent induced reduced progression of cell cycle and/or growth arrest, whereas the host cells have not. If the cells are cultured for a period long enough for the cell genotypes of the host cell (i.e. the reference cell) to start dividing, the potentially beneficial genotypes of the variant cell library exhibiting increased progression of cell cycle can no longer be selected over the genotypes of the reference host cells. Despite the adjusted cultivation period, in some embodiments, a very low number of the host cells, i.e. the reference cells, may have started to divide, for example, due to spontaneous mutations that have taken place in the genome of the individual reference cells.

In an embodiment, the selection of the cell genotypes of the variant cell library at the step (iii.1) is done before the genotypes of host cell (i.e. the reference cells) are optically detectable. For example, the optical detection is done by detecting cell colonies on agar plates, and the selection of the cell colonies of the variant cell library is done before the colonies of the host cell are detectable on the agar plate. In an embodiment, the selection of the cell genotypes of the variant cell library at the step (iii.1) is done when the cell genotypes of the variant cell library exhibit increased metabolic activity, but the reference cells do not.

In an embodiment, the cultivating of the step (ii) comprises cultivating separately, in the presence of the sulfhydryl reagent:
- the cells comprising a variant cell library, thereby obtaining cell genotypes of the variant cell library, and
- the cells provided at the step (i) without the variant cell library, thereby obtaining cell genotypes of the host cell;
and wherein the selection of the step (iii) comprises selecting the cell genotypes of the variant cell library exhibiting increased progression of cell cycle when compared to the cell genotypes of the host cell, thereby obtaining the subgroup comprising cells having increased production of the thiol-containing compound(s).

In an embodiment, the selecting of cell genotypes of the variant cell library exhibiting increased progression of cell cycle at the step (iii.1) means, the cell genotypes of the variant cell library which continue the cell cycle and start to divide before the cell genotypes of the host cell, are selected.

In an embodiment, the growth arrest of the selected cell genotypes of the variant cell library is shorter, as said cells require a shorter time to convert the sulfhydryl reagent within the growth environment of the cells and continue the cell cycle. In an embodiment, the growth arrest of the selected cell genotypes of the variant cell library is shorter, as said cells have an increased production the thiol-containing compound, and hence, require a shorter time period to convert and remove the sulfhydryl reagent, such as diamide, from the growth environment of the cells and continue the cell cycle.

In an embodiment, the step (ii.1) of the method comprises the host cells and the variant cell library are cultivated separately from each other. In an embodiment, the step (ii.1) of the method comprises the individual host cells and the individual cells of the variant cell library are cultivated separately from each other, thereby allowing the selection of cells exhibiting increased progression of cell cycle when compared to the rest of the cultivated cells. For example, the cells are cultivated on an agar plate, each individual cell (genotype) thereby forming its own separate colony, i.e. the genotypes are cultivated separately from each other. In another embodiment, the cells are cultivated in liquid medium in lipid droplets or alginate beads, each individual genotype of a cell thereby being cultivated separately from each other in their own droplet or bead. In an embodiment, the cell genotypes of the host cell all have nearly the same genotype with only minor/insignificant alterations, as the cell genotypes of the host cell have not been exposed to mutagenesis of the step (i.2).

In an embodiment, the current screening method comprises the steps of:
(i) providing host cells by:
   - introducing into a host cell genetic elements encoding an amino acid sequence comprising at least one cysteine residue, or
   - providing a host cell comprising genetic elements encoding an amino acid sequence comprising at least one cysteine residue;
(i.2) introducing into the host cell mutagenesis, thereby obtaining a variant cell library;
(ii.1) cultivating separately, in the presence of a sulfhydryl reagent
   - the host cell, thereby obtaining cell genotypes of the host cell, and
   - the variant cell library, thereby obtaining cell genotypes of the variant cell library; and
(iii.1) selecting cell genotypes of the variant cell library exhibiting increased progression of cell cycle when compared to the cell genotypes of the host cell, thereby obtaining a first set of mutant cell genotypes which is the subgroup comprising cells having increased production of the thiol-containing compound(s).

In an embodiment, the host cells provided at the step (i) comprise the genetic elements encoding an amino acid sequence comprising at least one cysteine residue, and the method comprises the step (i.2) wherein mutagenesis is introduced into the host cell thereby obtaining a variant cell library, and the reference cells at the cultivation step (ii.1), based on which the selection of the step (iii.1) is made, comprise the host cells comprising the genetic elements encoding an amino acid sequence comprising at least one cysteine residue. In such an embodiment, the duration of the cultivation step (ii.1) is selected so, that only some of the cell genotypes of the variant cell library have started exhibit increased progression of cell cycle, i.e. have overcome the sulfhydryl reagent induced reduced progression of cell cycle and/or growth arrest, whereas the reference cells (the host cells comprising the genetic elements encoding an amino acid sequence comprising at least one cysteine residue) have not.

In the following, the cultivation step (iv) which comprises cultivating as separate genotypes, in the presence of a sulfhydryl reagent the host cell, thereby obtaining separate genotypes of the host cell, and the mutant cells of the first set of mutant cell genotypes, is marked as a step (iv.1), and the selection step (v) which comprises selecting separate genotypes of the first set of mutant cell genotypes exhibiting increased progression of cell cycle when compared to the separate genotypes of the host cell, is marked as a step (v.1). Nevertheless, what is described for the steps (iv) and (v), applies also for the steps (iv.1) and (v.1), respectively. Thus, the steps (iv) and (iv.1); and the steps (v) and (v.1) may be used interchangeably.

In an embodiment, the method comprises the steps:
(iv.1) cultivating as separate genotypes, in the presence of a sulfhydryl reagent
   - the host cell, thereby obtaining separate genotypes of the host cell, and
   - the mutant cells of the first set of mutant cell genotypes of the step (iii.1), thereby obtaining separate genotypes of the first set of mutant cell genotypes; and
(v.1) selecting separate genotypes of the first set of mutant cell genotypes exhibiting increased progression of cell cycle when compared to the separate genotypes of the host cell, thereby obtaining a second set of mutant cell genotypes, which is the second subgroup.

In an embodiment, the step (iv.1) of the method comprises the host cells, which comprise the genetic elements encoding an amino acid sequence comprising at least one cysteine residue. In an embodiment, cultivating as separate genotypes at the step (iv.1) means that each host cell and the mutant cell of the first set of mutant cell genotypes is cultivated separately from each other, optionally on separate culture vessels. In an embodiment, the step (iv.1) of the method comprises cultivating as separate genotypes, in the presence of a sulfhydryl reagent such as diamide, the cells of the first set of mutant cell genotypes of the step (iii.1), thereby obtaining separate genotypes of the first set of mutant cell genotypes.

In an embodiment, the cultivation of the step (ii.1) is done according to the cultivation step (ii.1). In an embodiment, the reference cells at the cultivation step (iv.1), based on which the selection of the step (v.1) is made, comprise the separate cell genotypes of the host cell cultivated at the step (iv.1). In an embodiment, the selection step (v.1) is done according to the selection step (iii.1). In an exemplary embodiment, the step (iii.1) of the process comprises selecting cell genotypes of the variant cell library by inoculating cell colonies from solid agar plates, and step (iv.1) of the process comprises cultivating said inoculated cell colonies as separate genotypes in liquid growth medium, separately from the host cells comprising genetic elements encoding an amino acid sequence comprising at least one cysteine residue.

In an embodiment, selecting separate genotypes of the first set of mutant cell genotypes exhibiting increased progression of cell cycle at the step (v.1) means the separate genotypes of the first set of mutant cell genotypes which start to divide before the cell genotypes of the host cell, are selected.

In an embodiment, the method comprises screening for cell genotypes of the variant library wherein a mutation introduced at the mutagenesis increases the production of thiol-containing compounds of said cell genotypes of the variant library. In an embodiment, the method comprises screening for cell genotypes of the variant cell library wherein a mutation introduced at the mutagenesis increases the protein production of said cell genotypes of the variant cell library.

In an embodiment, the screening method screens for genotypes of the variant cell library having increased production of the thiol-containing compound(s) when compared to the cell genotypes of the host cell. In an embodiment, the screening method screens for increased overall protein production of genotypes of the variant cell library when compared to the genotypes of the host cell.

In an embodiment, the protein production can be assessed with any known method in the art, including, but not being limited to, immunoassays, methods based on fluorescent signal, western blotting, mass spectrometry, any activity measurement, and/or any affinity binding assay.

In an embodiment, the method further comprises the step(s):
(iii.2) assessing production of the thiol-containing compound(s) of the cells of the subgroup, and selecting cells having an increased production of the thiol-containing compound(s) when compared to:
   - the other cultivated cells of the step (ii), and/or
   - the other cells of the subgroup of the step (iii);
      and/or
(vi) assessing production of the thiol-containing compound(s) of the cells of the second subgroup, and selecting cells having an increased production of the thiol-containing compound(s) when compared to:
   - the other cultivated cells of the step (iv), and/or
   - the other cells of the second subgroup of the step (v).

In an embodiment, assessing production of the thiol-containing compound(s) refers to assessing protein production including secretion. In an embodiment, protein production including secretion is assessed at the step (iii.2) and/or the step (vi). In an embodiment, the expression level of the amino acid sequence comprising at least one cysteine residue is assessed at the step (iii.2) and/or the step (vi). In an embodiment, assessing protein production means quantifying the production of the produced proteins.

In an embodiment, the step (iii.2) comprises assessing production of the thiol-containing compound(s) of the cells of the subgroup. In an embodiment, the step (iii.2) comprises assessing the protein production of the cells of the subgroup. In an embodiment, the step (iii.2) comprises assessing the production of the amino acid sequence comprising at least one cysteine residue of the cells of the subgroup. In an embodiment, the step (iii.2) comprises selecting cells having an increased production of the thiol-containing compound(s), such as the amino acid sequence comprising at least one cysteine residue, when compared to the other cultivated cells of the step (ii), and/or when compared to the other cells of the subgroup.

In an embodiment, the cells of the subgroup assessed at the step (iii.2) are the cells comprising the genetic elements encoding an amino acid sequence comprising at least one cysteine residue, obtained from the step (i.a) or at the step (i.b).

In an embodiment, the cells of the subgroup assessed at the step (iii.2) are the cells of the first set of mutant cell genotypes of the variant cell library obtained from the step (i.2).

In an embodiment, the step (iii.2) of the current screening method comprises selecting cell genotypes of the variant cell library having an increased production of the thiol-containing compound(s), when compared to the cell genotypes of the host cells without the variant cell library cultivated at the step (ii), and/or when compared to the other genotypes of the variant cell library cultivated at the step (ii.1).

In an embodiment, the step (iii.2) of the current screening method comprises assessing the production of the thiol-containing compound. In an embodiment, the step (iii.2) of the current screening method comprises assessing the protein production of the cells of the subgroup and selecting cells having increased protein production when compared to the reference cells cultivated at the step (ii), or at the step (ii.1), and/or to the other cells of said subgroup. In an embodiment, the step (iii.2) of the current screening method comprises assessing the protein production of the cells of the subgroup, said cells comprising the genetic elements encoding an amino acid sequence comprising at least one cysteine residue, and selecting cells having increased protein production when compared to the host cell without said genetic elements. In an embodiment, the step (iii.2) of the current screening method comprises assessing the protein production of the cells of the first set of mutant cell genotypes obtained from the variant cell library at the step (iii.1) and selecting the genotypes having increased protein production when compared to the cell genotypes of the host cell and to the other cells of the first set of mutant cell genotypes. In an embodiment, the step (iii.2) of the current screening method comprises assessing the production of the thiol-containing compound, which is, for example, a metabolite, thiolalcohol, or a free cysteine residue.

In an embodiment, the step (vi) comprises assessing production of the thiol-containing compound(s) of the cells of the second subgroup. In an embodiment, the step (vi) comprises assessing the protein production of the cells of the second subgroup. In an embodiment, the step (vi) comprises assessing the production of the amino acid sequence comprising at least one cysteine residue of the cells of the second subgroup. In an embodiment, the step (vi) comprises selecting cells having an increased production of the thiol-containing compound(s), such as the amino acid sequence comprising at least one cysteine residue, when compared to the reference cells cultivated at the step (iv), and/or when compared to the other cells of the second subgroup. In an embodiment, the step (vi) of the current screening method comprises assessing the protein production of the cells of the second subgroup and selecting cells having increased protein production when compared to the reference cells cultivated at the step (iv), or at the step (iv.1), and/or to the other cells of said second subgroup. In an embodiment, the step (vi) comprises assessing the protein production of the cells of the second subgroup, said cells comprising the genetic elements encoding an amino acid sequence comprising at least one cysteine residue, and selecting cells having increased protein production when compared to the host cell without said genetic elements.

In an embodiment, the cells of the second subgroup assessed at the step (vi) are cell genotypes of the variant cell library. The step (vi) which comprises assessing production of the thiol-containing compound(s) of the mutant cells of the second set of mutant cell genotypes and selecting candidate mutant cells having increased production of the thiol-containing compound(s) when compared to the other cultivated cells of the step (iv.1), and/or when compared to the other cells of the second set of mutant cell genotypes, is marked as a step (vi.1). Nevertheless, what is described for the steps (vi) applies also for the step (vi.1). Thus, the steps (vi) and (vi.1) may be used interchangeably.

In an embodiment, the step (vi.1) comprises assessing protein production of the mutant cells of the second set of mutant cell genotypes and selecting candidate mutant cells having increased protein production when compared to the reference cells cultivated at the step (iv.1), and/or when compared to the other cells of the second set of mutant cell genotypes.

In an embodiment, the step (vi.1) of the method comprises assessing protein production of the mutant cells of the second set of mutant cell genotypes obtained from the step (v.1) and selecting candidate mutant cells having increased protein production when compared to the separate genotypes of the host cell cultivated at the step (iv.1).

In an embodiment, the step (vi.1) comprises selecting cell genotypes of the variant cell library having an increased production of the thiol-containing compound(s), when compared to the cell genotypes of the host cells without the variant cell library, cultivated at the step (iv.1), and/or when compared to the other genotypes of the variant cell library cultivated at the step (iv.1).

In an embodiment, the overall production of the thiol-containing compound, such as a protein, is assessed in the step (iii.2) and/or in the step (vi)/(vi.1). In an embodiment, the overall thiol-containing compound production of selected candidate cells is assessed in the step (iii.2) and/or in the step (vi)/(vi.1). In an embodiment, the cells selected in the step (iii)/(iii.1), and/or in the step (v)/(v.1) are the candidate cells. In an embodiment, the production of the amino acid sequence comprising at least one cysteine residue is assessed in the step (iii.2) and/or in the step (vi)/(vi.1).

In an embodiment, based on the assessment of the protein production, candidate cells are selected for further use or analysis. In an embodiment, the further analysis includes analysis of mutated sites in the selected cell's genome and/or analysis of the effect said mutated sites have on protein production. In an embodiment, a further use of a candidate cell comprises use in protein production.

In an embodiment, the method comprises identifying mutation sites in the genome of the cells of the (first) subgroup and/or in the cells of the second subgroup, after the selections step (iii) and/or the selection step (v). In an embodiment, the method comprises identifying mutation sites in the genome of the selected cells obtained from the step (iii.2) and/or from the step (vi). In an embodiment, the method comprises identifying a mutation site of the mutagenesis in the genome of the selected cell genotypes of the variant cell library, obtained from the step (iii.1) and/or from the step (v.1).

In an embodiment, the method comprises identifying mutation sites of spontaneous mutations which have taken place in the genome of the selected cells. In an embodiment, the method comprises identifying mutation sites of targeted mutations which have been introduced in the genome of the selected cells at the step (i.2). In an embodiment, the method comprises identifying mutation sites of random mutations which have been introduced in the genome of the selected cells at the step (i.2).

In an embodiment, wherein the variant cell library is obtained through random or targeted mutagenesis at the step (i.2), the method comprises identifying a mutation site of the mutagenesis in the genome of the cells of the first and/or second set of mutant cell genotypes, obtained from the step (iii.1) and/or the step (v.1), respectively.

In an embodiment, the identification of the mutation site in the genome of the candidate mutant cell can be identified with any suitable method known in the art, depending on the specific mutagenesis introduced to the host cells at the step (i.2). For example, wherein the variant cell library is obtained through random transposon mutagenesis, the insertion locus of the transposon can be determined using FPNI-PCR.

In an embodiment, after identifying a mutation site of the mutagenesis in the genome of the selected cell genotype, the effect of the identified mutation locus can be verified with a targeted mutation. In an embodiment, said effect of the identified mutation can be verified, for example, with seamless deletion to the host cell genome, to recreate the candidate mutant cell in question. In an embodiment, the production of a protein of interest, such as the amino acid sequence comprising at least one cysteine residue, can be verified in the cells comprising said targeted mutation.

In an embodiment, the final selection of the candidate cells having increased production of the thiol-containing compound when compared to the reference cells is first done after identifying a mutation site of mutagenesis in genome of selected cells, after the step (iii) and/or (v). In an embodiment, the final selection of the candidate mutant cells having increased production of the thiol-containing compound is first done after identifying a mutation site of the mutagenesis in said genome of the cells of the first set of mutant cell genotypes and/or in the cells of the second set of mutant cell genotypes, after the selections step (iii.1) and/or the selection step (v.1).

In an embodiment, the method comprises identifying a mutation site of the mutagenesis in the genome of the mutant cells of the first set of mutant cell genotypes after the step (iii.1) and selecting candidate mutant cells based on the mutation site analysis.

In an embodiment, the method comprises identifying a mutation site of the mutagenesis in the genome of the mutant cells of the second set of mutant cell genotypes after the step (v.1) and selecting candidate mutant cells based on the mutation site analysis.

In an embodiment, if in the mutation site analysis, the same gene is detected mutated in plurality of genotypes, the candidate mutant cells carrying such mutation are selected. In an embodiment, the selection at any one, or in all of the steps (iii), (iii.1), (v), and (v.1), includes selecting candidate mutant cells carrying a gene mutation which is separately detected more than once, preferably more than twice when identifying a mutation site of the mutagenesis in the genome of the candidate mutant cells.

In an embodiment, the step (iii.2) and/or the step (vi) comprises assessing the production of a protein of interest in the cells of the subgroup and/or the second subgroup.

In an embodiment, the step (iii.2) and/or the step (vi) comprises assessing the production of a protein of interest in the cells of the subgroup obtained from the step (iii) and/or the second subgroup obtained from the step (v), wherein the protein of interest is preferably the amino acid sequence comprising at least one cysteine residue. In an embodiment, the step (iii.2) and/or the step (vi) comprises assessing the production of a protein of which is another protein than the amino acid sequence comprising at least one cysteine residue. In an embodiment, the step (iii.2) and/or the step (vi) comprises assessing the production of a protein of interest, wherein the protein of interest comprises at least one cysteine residue.

In an embodiment, the step (iii.2) comprises assessing the production of a protein of interest in the mutant cells of the first set of mutant cell genotypes obtained from the step (iii.1). In an embodiment, the step (vi.1) comprises assessing the production of a protein of interest in the mutant cells of the second set of mutant cell genotypes obtained from the step (v.1). In an embodiment, the step (iii.2) and/or the step (vi.1) comprises assessing the production of a protein of interest in the cell genotypes of the variant cell library, wherein the protein of interest is preferably encoded by the genetic elements encoding the amino acid sequence comprising at least one cysteine residue. In an alternative embodiment, the step (iii.2) and/or the step (vi.1) comprises assessing the production of a protein of interest in the cell genotypes of the variant cell library, wherein the protein of interest is not encoded by the genetic elements encoding the amino acid sequence comprising at least one cysteine residue.

The current screening method provides fast and reliable screening method which is easily adjustable to many different host cells, and production proteins. The current screening method significantly reduces the number of genes to be screened for beneficial effects on protein production of selected host cells. For example, in an embodiment, wherein the host cell is B. *subtilis,* and the screening method comprises two different cultivation and two different selection steps, the number of genes to be assessed for their effect on protein production can be reduced at least 1/200, preferably at least 1/300, even more preferably at least 1/350, more preferably at least 1/380 from the total amount of genes present in the host cell.

The current screening method is further beneficial as it allows identifying potentially beneficial effects arising from mutations in the non-coding DNA sequence of the host cell. In an embodiment, the present method allows detecting beneficial effects arising from a mutation in in the non-coding DNA sequence. In an embodiment, the beneficial effects arising from a mutation in in the non-coding DNA sequence include, but are not limited to, increased protein production and stability.

### EXAMPLES

Various embodiments have been presented. It should be appreciated that in this document, words comprise, include, and contain are each used as open-ended expressions with no intended exclusivity.

The following examples are provided to illustrate various aspects of the present invention. They are not intended to limit the invention, which is defined by the accompanying claims.

### Example 1: Vector and cell construction

### Vector design

Unless otherwise stated, molecular biological methods including DNA manipulations and transformations were performed as described by Sambrook and Russell 2001 and Harwood 1990. A synthetic *Gaussia princeps* luciferase gene (*IucG;* Accession: AY015993) ordered at GENEWIZ Germany GmbH (Leipzig) was cloned into a pUB110 derivative. *Gaussia princeps* luciferase LucG encoded by said luciferase gene, contains 11 cysteines (Wu et al. 2015). The *lucG* gene was set under control of the *Pylb* promoter (Yu et al. 2015) and combined with the signal peptide of bacillopeptidase F Bpr (Accession P16397). Primers with overhangs between backbone and insert were designed (see Table 1), and the fragments were amplified using Phusion PCR according to the standard protocol. Following PCR conditions were used for plasmid construction: 120 sec initial denaturation at 94 °C, followed by 35 cycles of 15 sec at 94 °C, 30 sec annealing at one of the following 50/55 °C, 110/290 sec extension at 68 °C and the final extension at 68 °C for 10 min. The vector was constructed using Gibson assembly with NEBuilder HiFi DNA assembly (New England Biolabs GmbH, Frankfurt) following manufacturer's instruction. Cloning was performed in competent *B. subtilis* SCK6 cells (Zhang und Zhang 2011). The resulting plasmid was named pABES029 as shown in Fig. 1.

**Table 1: List of oligonucleotides used for cloning of Gaussia princeps luciferase lucG expression cassette in pUB110 derivate.**

| **Primer** | **Template** | **Sequence** |
|---|---|---|
| VEC001 | pUB110 derivative | AGAGATATACCGACAGTGTTTAAAGAG |
| VEC002 | pUB110 derivative | GTTTCCTCTCCCTCTCATTTTC |
| SEC0001 | lucG (synthetic gene) | AAATGAGAGGGAGAGGAAACATGAAACCAACTGA AAACAATGAAGATTTC |
| SEC0007 | lucG (synthetic gene) | AACACTGTCGGTATATCTCTTTAATCACCACCGG CACCCTTTATTTTG |
| SEC0027 | *Bacillus subtilis* 168 | AGCTCTGTTTTAAGTACAGTTGTCATCAGTTCACT GCTGTTTCCGGGAGCAGCCGGGGCAAAACCAAC TGAAAACAATG |
| SEC0028 | *Bacillus subtilis* 168 | ACTGATGACAACTGTACTTAAAACAGAGCTGATG AGTCTGTTTTTCGTTTTTTTCCTCATACGTTCTACC TTTGTCAAAC |
| SEC0015 | *Bacillus subtilis* 168 | ATAACAGGAATTCTCATGTTTGACAGCTTAACTTC TCAAAGATCCCATGTG |
| SEC0018 | *Bacillus subtilis* 168 | GAAATCTTCATTGTTTTCAGTTGGTTTCATACGTT CTACCTTTGTCAAACAAATC |

### The cell design

The plasmid pABES029 (Fig. 1) was transformed into strain *Bacillus subtilis* DB430 *ΔlipA* by natural competence (van Dijl et al. 1992). The transformed cells were plated on standard LB agar supplemented with tetracycline (10 µg/mL). Arising colonies were picked and verified by colony PCR and Sanger sequencing (Microsynth, Switzerland) to confirm plasmid integration and integrity, thereby obtaining the provided host cell.

### Example 2: Growth arrest caused by diamide supplementation

The host strain *Bacillus subtilis* DB30 *ΔlipA* and *Bacillus subtilis* DB30 Δ*lipA* containing the LucG expression plasmid were tested for their growth behavior under diamide stress conditions in 96 microtitre plates. Cultures of both strains were inoculated into 200 µL LB medium (Lennox, Carl-Roth, Karlsruhe) containing a range of diamide (Merck KGaA, Darmstadt) concentrations from 0 to 10 mM. Optical density at 600 nm, detecting cell division, was measured throughout growth during 18 h at 37 °C in a BioTek SynergyMx plate reader (Agilent, US) under constant shaking (medium speed, double orbital). The experiment revealed that the strain containing the LucG expression plasmid was able to leave the growth arrest caused by supplementation of diamide earlier than the strain without LucG expression plasmid in each diamide concentration between 0 - 0.5 mM (Fig. 2). Neither of the strains cultured in diamide concentrations of 10 mM, 5 mM and 1 mM was capable to overcome the diamide-induced growth arrest during the measuring period of 18 h. The expression of the *Gaussia princeps* luciferase LucG containing 11 cysteines considerably reduced the duration of the growth arrest in the presence of diamide compared to the strain without the cysteine containing LucG luciferase. The strongest effect, i.e. the largest difference in growth arrest duration between the host strain and the host strain containing the LucG expression plasmid, could be observed in LB medium supplemented with 0.5 mM diamide.

### Example 3: Screening method

### Variant cell library design and transposon mutagenesis

To generate a variant cell library, the method of random transposon mutagenesis was chosen. This method relies on the use of the pMarB plasmid which contains a *Himarl* C9 transposase gene under the control of a SigB dependent promoter and TnYLB-1 transposon (transposable element) (Le Breton et al. 2006). The transposon mutagenesis was performed as described in Le Breton et al. 2006 unless otherwise stated. In summary, the pMarB plasmid was added to the host cells (*Bacillus subtilis* DB30 *ΔlipA* containing the LucG expression plasmid) through a second round of transformation with plating on agar plate under tetracycline and erythromycin selection (5µg/mL tetracycline and 2 µg/mL erythromycin). Plasmid integration and integrity was again verified by PCR and Sanger sequencing (Microsynth, Switzerland). Sequence comparisons were done using ClustalW sequence alignment Thompson et al. 1994.

Colonies were formed on the plate with *B. subtilis* DB430 *ΔlipA* (containing the plasmids pABES029 and pMarB), which were utilized in further. An overnight culture in baffled 100 mL shake flask containing 10 mL LB medium with 2 µg/mL erythromycin and 5 µg/mL tetracycline was inoculated from the colonies on the plate with *B. subtilis* DB430 Δ*lipA* containing the plasmids pABES029 and pMarB. The overnight culture was grown at 28 °C and 180 rpm orbital shaking in an incubator for 16 h (Multitron II, Infors HT, Switzerland). The following day a fresh 100 mL baffled shake flask containing 10 mL LB without antibiotic was inoculated to an O.D.₆₀₀ of 0.1 from the overnight culture. This shake flask was cultivated at 37 °C and 180 rpm orbital shaking in an incubator (Multitron II, Infors HT, Switzerland) for 5 h to reduce the plasmid copy number in the cells. The cells were then submitted to a 30-minute heat shock in a 50 °C water bath to induce the expression of the *Himarl* C9 transposase. Inside a cell, transposase cuts out the TnYLB-1 transposon from the plasmid, which is then inserted randomly into the genome at a suitable location (base pair sequence AT), thereby obtaining the variant cell library.

Cells were subsequently plated (100 µL undiluted cell suspension per plate) on LB agar plates containing 200 µM diamide and 10 µg/mL kanamycin. The plates were then cultivated in the presence of diamide for 40 h, and thereby cell genotypes of the variant cell library were obtained.

Subsequently, a second round of screening was performed to selected set (i.e. first set) of mutant cell genotypes in microtiter plates, to identify valuable candidate mutant cells.

### Kinetic diamide growth assay

An overnight culture was prepared in a 96-deepwell (2 mL) plate (Merck KGaA, Darmstadt). The wells on the 96-deepwell plate were filled with 1 mL fresh LB medium and inoculated with selected cell genotypes of the variant cell library (previously cultivated on the LB agar plates in the presence of 200 µM diamide for 40 h). In addition, three wells were inoculated with *B. subtilis* DB430 *ΔlipA* cells containing the pABES029 expression plasmid, without transposon mutagenesis which was included as a reference strain. The plate was then sealed with a semi-permeable membrane and cultivated at 37 °C and 800 rpm orbital shaking in an incubator (Multitron II, Infors HT, Switzerland). The following day a sterile transparent 96-well microtiter plate (Greiner Cellstar, Merck KGaA, Darmstadt) was prepared with 200 µL LB supplemented with 2mM diamide in each well. The wells were inoculated with 10 µL overnight culture of selected separate mutant cell genotypes (i.e. colonies) from the corresponding well from the 96-deepwell plate. The microtiter plate was placed in a plate reader (BioTek Synergy Mx) preheated at 37 °C. The plate was incubated under constant shaking and the absorption at 600 nm was measured at 10 min intervals for the duration of 30 h. The overnight 96-deepwell plates were frozen at -80 °C after inoculation of the kinetic diamide assay.

As diamide is sensitive to light and heat, the reference strain was included during each microtiter plate experiment to eliminate the effect of diamide degradation during assay preparation. Using the measurements of this reference strain, a confidence interval could be established to distinguish between natural variation in the growth arrest duration within the reference strain, and candidate mutant cells with a desirable phenotype.

The data received from optical density measurement at 600 nm was analyzed with computational methods. An O.D.₆₀₀ value of 0.5 was chosen as a threshold value, above which it is assumed growth of cells had resumed, thereby establishing standard growth delay (i.e. standard duration of growth arrest) for the reference cells. Using the time at which a reference strain sample reached said value of 0.5, further calculations could be made, and a confidence interval for the standard growth delay could be established according to Harris et al., 2020.

The results of the kinetic diamide growth assay analysis are shown in Figure 3. The standard growth delay of the reference cell strain is shown as a solid line in Fig. 3. The confidence interval is shown as dashed lines on both sides of the standard growth delay. The individual mutant cell genotypes falling below the lower confidence interval at 25 h growth delay (candidate cells exhibiting shortened growth arrest) were selected, making up the second set of mutant cell genotypes. These mutant cell genotypes were considered as beneficial candidate cells comprising potentially beneficial mutations inducing protein production and/or stability.

### Example 4: mutation site identification and strain evaluation

### FPNI PCR

After selection of beneficial candidate cells from the second set of mutant cell genotypes by the kinetic diamide growth assay, transposon integration sites were identified by applying FPNI (fusion primer and nested integrated) PCR. FPNI-PCR was utilized to produce fragments containing a specific primer-targeted part of the transposon and the adjacent genomic locus. In summary, the principle of the FPNI-PCR method is using in a first PCR reaction a degenerate primer which randomly binds to a genome, and a primer that specifically binds on the transposon. This first reaction product is then used as input for a second PCR reaction which uses specific primers binding to the degenerate primer and the transposon. The FPNI PCR was performed as described in Wang et al 2011. Oligonucleotides used for detection are listed in table 2.

**Table 2: Oligonucleotides used for FPNI PCR**

| **Primer** | **Sequence** |
|---|---|
| 2step_3 | ACAAGTGGTATGACATTGCC |
| 2step_4 | CGAAGAGGAACTTGTCTTTTCCC |
| 2step_seq_3 | AAGTTCGCTAGATAGGGGTCC |
| FAP_1 | GCGTTCAACTTTGGGAGAGACTGGC |
| pAL13_sso_4 | GCGTTCAACTTTGGGAGAG |

The FPNI-PCR was confirmed by using gel electrophoresis with a 1 % agarose gel in TAE buffer (40 mM Tris-acetate, 1 mM EDTA) containing Roti-Safe Gelstain (Carl Roth, Karlsruhe). The gel was run for 30 minutes at 8 V/cm after which the bands were visualized using UV-light. The successful FPNI-PCRs were then purified using spin columns from Wizard SV Gel and PCR Clean-up system (Promega, USA). From the purified PCR product, 12 µL were mixed with 3 µL primer 2step_seq_3 (10 µM) and sent for Sanger sequencing (Microsynth, Switzerland). As a result, a total of over 70 unique gene disruptions could be identified according to the described screening method. Details of seven exemplary gene disruptions numbered as genes 1-7 are shown in the table 3. Furthermore, four genes out of the over 70 identified genes of interest were selected for targeted disruption to confirm the gene knockout effects on protein production and growth arrest. These selected four genes were gene disruptions that were either detected multiple times in the sequencing results of the described screening method, or gene disruptions showing the most promising results. The selected four genes, numbered as genes 2, 3, 4, and 6, are included in the table 3 and shown underlined.

**Table 3: Notable gene disruptions identified from FPNI-PCR and Sanger sequencing. Selected four genes shown underlined.**

| Gene | Comment |
|---|---|
| No.1 | Locus disrupted in 2 separate candidate cells |
| No. 2 | Locus disrupted in 9 separate candidate cells |
| No. 3 | Locus disrupted in 5 separate candidate cells |
| No. 4 | Locus disrupted in 3 separate candidate cells |
| No. 5 | Highest measured reduction in growth delay (-70%) |
| No. 6 | Locus disrupted in 2 separate candidate cells |
| No. 7 | Locus disrupted in 2separate candidate cells |

### Seamless gene deletion

A seamless gene deletion strategy was employed to verify the effect of the identified mutation loci received for the candidate mutant cells by the screening method. The applied method is based on the protocol and vector system described in Rachinger et al. 2013. For deletion of the selected four genes, 500 bp regions up- and downstream from the selected four genes were amplified from extracted genomic DNA (DNA blood & tissue kit, Qiagen, Hilden) of the reference cell strain using Phusion PCR (New England Biolabs GmbH, Frankfurt). The amplified products (amplicons) contained a 25-30 bp overlap with a pKVM2 deletion vector on one side of the amplicon, and a 25-30 bp overlap with the other amplified fragment on the other side of the amplicon. The amplified products were combined to the pKVM2 backbone using 3-part Gibson assembly (NEBuilder HiFi DNA assembly, New England Biolabs GmbH, Frankfurt). The deletion vector reaction mixtures, prepared according to the protocol, were incubated at 50 °C for 1 h before being transformed into competent *B. subtilis* SCK6 cells (Zhang und Zhang 2011). These cells were incubated for 1.5 h at 37 °C before being plated on LB agar plates supplemented with tetracycline (10 µg/mL). The plates were then incubated at 28 °C since the pKVM2 plasmid has a temperature sensitive origin of replication. Integration and integrity of the deletion vector was verified by colony PCR and sanger sequencing on isolated plasmid preparations.

The four different deletion vectors were then transformed using natural competence into the target strain *B. subtilis* DB430 *ΔlipA.* To integrate the plasmid into the genome, an overnight culture of the target strain in LB with tetracycline 10 µg/mL was inoculated and grown with 180 rpm orbital shaking at 28 °C. The following day, a 10-fold dilution series was made and 100 µL dilutions were plated on LB agar plates with tetracycline. These plates were incubated at 42 °C until growth of cell colonies was observed. The presence of the plasmid was verified by colony PCR.

Next the chromosomal insertion of the plasmid was reverted by cultivation of the cells in LB medium in the absence of antibiotic selection. A dilution series of the cells was made and plated on LB plates containing X-gal. Cells that lost the plasmid remained white due to the absence of the *bgaB* gene located on the plasmid. Positive clones were identified by colony PCR.

### Gene deletion strain evaluation

The pABES029 luciferase expression vector was introduced to the four different *B. subtilis* DB430 *ΔlipA* gene deletion mutants (described above) through transformation by natural competence. Presence of the pABES029 plasmid in the cells was verified by colony PCR. Overnight cultures of the four pABES029-carrying gene deletion mutants, and of the reference strain were prepared in a 96-deepwell (2 mL) plate: the wells were filled with 1 mL fresh LB medium and inoculated in triplicates with colonies of the four pABES029-carrying gene deletion mutants and the *B. subtilis* DB430 *ΔlipA* reference cell strain, all expressing the *Gaussia princeps* luciferase (i.e. comprising the pABES029 luciferase expression vector). The plate was then sealed with a semi-permeable membrane and grown at 37 °C and 800 rpm orbital shaking in an incubator (Multitron II, Infors HT, Switzerland) overnight or 16 - 18 h. The following day a sterile transparent 96-well microtiter plate was prepared with 200 µL LB per well to which 10 µL of the overnight culture was added. The plate was incubated at 37 °C with 800 rpm for 6 h in an incubator (Multitron II, Infors HT, Switzerland). To measure the amount of active *Gaussia princeps* luciferase produced in cultures, an enzyme activity assay was used. Supernatant from the *B. subtilis* DB430 *ΔlipA* cell cultures was used directly to perform the *Gaussia princeps* luciferase assay (Pierce^{™} *Gaussia* Luciferase Glow Assay Kit, Thermo Fisher Scientific). 20 µL of culture supernatant was supplemented into the wells of an opaque microtiter plate, after which 50 µL of assay reaction mix was added per well. The plate was shaken and left to equalize for 10 minutes before luminescence was measured at 480 nm in a microplate reader (Clariostar, BMG Labtech). A blank reaction was prepared with pure LB medium as a control.

The selected four different deletion mutant cells showed significantly higher luciferase activity in their culture supernatant than the reference strain. The assay result can be seen in Fig. 4. The results show the current screening method efficiently screens for beneficial mutations improving protein production of the host cells.

The foregoing description has provided by way of non-limiting examples of particular implementations and embodiments a full and informative description of the best mode presently contemplated by the inventors for carrying out the invention. It is however clear to a person skilled in the art that the invention is not restricted to details of the embodiments presented in the foregoing, but that it can be implemented in other embodiments using equivalent means or in different combinations of embodiments without deviating from the characteristics of the invention.

Furthermore, some of the features of the afore-disclosed example embodiments may be used to advantage without the corresponding use of other features. As such, the foregoing description shall be considered as merely illustrative of the principles of the present invention, and not in limitation thereof. Hence, the scope of the invention is only restricted by the appended patent claims.

### References:

Harwood, C.R (Hg.) (1990): Molecular biological methods for bacillus. Chichester: Wiley & Sons (Modern microbiological methods).
Le Breton, Yoann; Mohapatra, Nrusingh Prasad; Haldenwang, W. G. (2006): In vivo random mutagenesis of Bacillus subtilis by use of TnYLB-1, a mariner-based transposon. In: Applied and Environmental Microbiology 72 (1), S. 327-333. DOl: 10.1128/AEM.72.1.327-333.2006.
Rachinger, Michael; Bauch, Melanie; Strittmatter, Axel; Bongaerts, J.; Evers, Stefan; Maurer, Karl-Heinz et al. (2013): Size unlimited markerless deletions by a transconjugative plasmid-system in Bacillus licheniformis. In: Journal of Biotechnology 167 (4), S. 365-369. DOI: 10.1016/j.jbiotec.2013.07.026.
Sambrook, Joseph; Russell, David W. (2001): Molecular cloning. A laboratory manual. 3. Aufl. 3 Bände. Cold Spring Harbor, N.Y: Cold Spring Harbor Laboratory Press (CSHL Press) (1 - 3).
Thompson, Julie D.; Higgins, Desmond G.; Gibson, Toby J. (1994): CLUSTAL W. Improving the sensitivity of progressive multiple sequence alignment through sequence weighting, position-specific gap penalties and weight matrix choice. In: Nucleic Acids Research 22 (22), S. 4673-4680. DOI: 10.1093/nar/22.22.4673.
van Dijl, J. M.; Jong, A. de; Vehmaanperä, J.; Venema, G.; Bron, S. (1992): Signal peptidase I of Bacillus subtilis: patterns of conserved amino acids in prokaryotic and eukaryotic type I signal peptidases. In: EMBO J. 11 (8), S. 2819-2828.
Wang, Zhen; Ye, Shafei; Li, Jingjing; Zheng, Bo; Bao, Manzhu; Ning, Guogui (2011): Fusion primer and nested integrated PCR (FPNI-PCR): a new high-efficiency strategy for rapid chromosome walking or flanking sequence cloning. In: BMC biotechnology 11, S. 109. DOl: 10.1186/1472-6750-11-109.
Wu, Nan; Rathnayaka, Tharangani; Kuroda, Yutaka (2015): Bacterial expression and re-engineering of Gaussia princeps luciferase and its use as a reporter protein. In: Biochim Biophys Acta 1854 (10 Pt A), S. 1392-1399. DOI: 10.1016/j.bbapap.2015.05.008.
Yu, Xiaoxia; Xu, Jiangtao; Liu, Xiaoqing; Chu, Xiaoyu; Wang, Ping; Tian, Jian et al. (2015): Identification of a highly efficient stationary phase promoter in Bacillus subtilis. In: Scientific Reports 5, S. 18405. DOI: 10.1038/srep18405.
Zhang, Xiao-Zhou; Zhang, Y-H Percival (2011): Simple, fast and high-efficiency transformation system for directed evolution of cellulase in Bacillus subtilis. In: Microbial Biotechnology 4 (1), S. 98-105. DOI: 10.1111/j.1751-7915.2010.00230.x.
Harris, C.R., Millman, K.J., van der Walt, S.J. et al. Array programming with NumPy. Nature 585, 357-362 (2020). DOI: 10.1038/s41586-020-2649-2. (Publisher link)

## Claims

1. A screening method for cells having increased production of a thiol-containing compound, the method comprising the steps of:
(i) providing cells producing the thiol-containing compound;
(ii) cultivating the cells separately from each other in the presence of a sulfhydryl reagent; and
(iii) selecting cells exhibiting increased progression of cell cycle when compared to the rest of the cultivated cells, thereby obtaining a subgroup comprising cells having increased production of the thiol-containing compound.

2. The method of claim 1, wherein the thiol-containing compound produced by the cells is selected from:
- an amino acid sequence comprising at least one cysteine residue;
- a free cysteine residue;
- a thiol-containing metabolite;
- a thiolalcohol; or
- any combination thereof.

3. The method of claim 2, wherein the thiol-containing compound is an amino acid sequence comprising at least one cysteine residue, and it is provided in the cells of the step (i) by:
(i.a) introducing into the cells genetic elements encoding an amino acid sequence comprising at least one, preferably at least 3, more preferably at least 5, even more preferably at least 10 cysteine residues, or
(i.b) providing the cells comprising genetic elements encoding an amino acid sequence comprising at least one, preferably at least 3, more preferably at least 5, even more preferably at least 10 cysteine residues.

4. The method of claim 3, wherein the amino acid sequence comprising at least one cysteine residue is provided in the cells of the step (i) with the alternative (i.a), and wherein less than 2 %, preferably less than 1 % of the amino acids comprised by secreted proteins of the cells are cysteines prior to providing said amino acid sequence comprising at least one cysteine residue into the cells.

5. The method of any preceding claim, wherein the thiol-containing compound is configured to be secreted by the cells provided at the step (i).

6. The method of any preceding claim, wherein conditions for the cultivating at the step (ii) are selected from:
- a sulfhydryl reagent concentration effective to cause reduced progression of cell cycle or cell cycle arrest in the cultivated cells;
- cultivation takes place on a solid growth medium;
- cultivation takes place in a liquid growth medium; or
- any combinations thereof.

7. The method of any preceding claim, wherein the sulfhydryl reagent is selected from diamide; 5,5'-dithiobis; DTNB; N,N,N',N'-tetramethyldiazene-1,2-dicarboxamide; p-chloromercuribenzoate; N-methylmaleimide; or any combination thereof, preferably the sulfhydryl reagent comprises diamide.

8. The method of any preceding claim, wherein the method further comprises:
(iv) cultivating in the presence of a sulfhydryl reagent the cells of the subgroup from the step (iii), thereby obtaining cell genotypes of the cells of the subgroup; and
(v) selecting cell genotypes exhibiting increased progression of cell cycle when compared to the rest of the cell genotypes of the subgroup, thereby obtaining a second subgroup comprising cells having increased production of the thiol-containing compound(s).

9. The method of claim 8, wherein the selecting of cells exhibiting increased progression of cell cycle at the step (iii) and/or at the step (v) is based on:
- optically detectable phenotypic selection, preferably the selection comprises selecting cells which are optically detectable earlier when compared to the rest of the cells;
- a measurement of metabolic activity, or
- any combinations thereof.

10. The method of claim 8 or 9, wherein
- the subgroup obtained at the step (iii) comprises cells having increased production of the thiol-containing compound(s) compared to the rest of the cultivated cells of the step (ii), and/or
- the second subgroup obtained at the step (v) comprises cells having increased production of the thiol-containing compound(s) compared to the rest of the cultivated cells of the step (iv).

11. The method of any preceding claim, wherein the cells of the step (i) comprise a variant cell library, wherein the variant cell library is preferably obtained by:
- introducing into the cells random mutagenesis;
- introducing into the cells targeted mutagenesis;
- introducing into the cells targeted mutagenesis thereby obtaining cell variants of a gene of interest; or
- any combinations thereof.

12. The method of claim 11, wherein the cultivating of the step (ii) comprises cultivating separately, in the presence of the sulfhydryl reagent:
- the cells comprising a variant cell library, thereby obtaining cell genotypes of the variant cell library, and
- the cells provided at the step (i) without the variant cell library, thereby obtaining cell genotypes of the host cell;
and wherein the selection of the step (iii) comprises selecting the cell genotypes of the variant cell library exhibiting increased progression of cell cycle when compared to the cell genotypes of the host cell, thereby obtaining the subgroup comprising cells having increased production of the thiol-containing compound(s).

13. The method of claim 11 or 12, comprising screening for cell genotypes of the variant cell library wherein a mutation introduced at the mutagenesis increases the protein production of said cell genotypes of the variant cell library.

14. The method of any one of claims 8 -13, further comprising the step(s):
(iii.2) assessing production of the thiol-containing compound(s) of the cells of the subgroup, and selecting cells having an increased production of the thiol-containing compound(s) when compared to:
- the other cultivated cells of the step (ii), and/or
- the other cells of the subgroup of the step (iii);
and/or
(vi) assessing production of the thiol-containing compound(s) of the cells of the second subgroup, and selecting cells having an increased production of the thiol-containing compound(s) when compared to:
- the other cultivated cells of the step (iv), and/or
- the other cells of the second subgroup of the step (v).

15. The method of claim 14, wherein the step (iii.2) and/or the step (vi) comprises assessing the production of a protein of interest in the cells of the subgroup and/or the second subgroup.
